# EUROPÄISCHE PATENTSCHRIFT

(11) **EP 2 459 588 B1**
(45) Veröffentlichungstag und Bekanntmachung des Hinweises auf die Patenterteilung: **29.06.2016**
(21) Anmeldenummer: 09776017.7
(22) Anmeldetag: 27.07.2009
(51) Int. Cl.: C07K 14/33, C07K 16/12, G01N 33/569

(54) **VERFAHREN ZUM NACHWEIS UND ZUR IDENTIFIKATION EINES VARIANTEN C. DIFFICILE STAMMES IN EINER PROBE**
METHOD FOR DETECTING AND IDENTIFYING A VARIANT C. DIFFICILE STRAIN IN A SAMPLE
PROCÉDÉ DE DÉTECTION ET D'IDENTIFICATION D'UNE VARIANTE DE SOUCHE DE C. DIFFICILE DANS UN ÉCHANTILLON& xA;

(43) Veröffentlichungstag der Anmeldung: 06.06.2012
(73) Patentinhaber: BioDics GmbH, 55444 Schweppenhausen (DE)
(72) Erfinder: VON EICHELSTREIBER, Christoph, 55444 Schweppenhausen (DE); MOOS, Michael, 69115 Heidelberg (DE); GISCH, Karina, 55131 Mainz (DE); BRAUN, Veit, 55122 Mainz (DE)
(74) Vertreter: Rudolph, Ulrike
(86) Internationale Anmeldenummer: PCT/DE2009/001044
(87) Internationale Veröffentlichungsnummer: WO 2011/012098

(56) Entgegenhaltungen:
- EP-A1- 0 153 519
- WO-A1-91/18293
- WO-A2-99/12971
- WO-A2-2006/121422
- DE-A1-102008 029 688
- US-A- 4 879 218
- HUANG HAIHUI ET AL: "Clostridium difficile infections in a Shanghai hospital: antimicrobial resistance, toxin profiles and ribotypes." INTERNATIONAL JOURNAL OF ANTIMICROBIAL AGENTS APR 2009, Bd. 33, Nr. 4, April 2009 (2009-04), Seiten 339-342, XP002570296 ISSN: 1872-7913
- RUPNIK MAJA: "Heterogeneity of large clostridial toxins: importance of Clostridium difficile toxinotypes." FEMS MICROBIOLOGY REVIEWS MAY 2008, Bd. 32, Nr. 3, Mai 2008 (2008-05), Seiten 541-555, XP002570297 ISSN: 0168-6445
- VON EICHEL-STREIBER C ET AL: "The difficile clostridium" LABORATORIUMS MEDIZIN / JOURNAL OF LABORATORY MEDICINE, WALTER DE GRUYTER GMBH & CO. KG, KIRCHHEIM, DE, Bd. 32, Nr. 4, 1. Januar 2008 (2008-01-01) , Seiten 219-234, XP009129993 ISSN: 0342-3026
- COUGHLIN R T ET AL: "Characterization of six murine monoclonal antibodies specific for toxin B of Clostridium difficile" HYBRIDOMA, LIEBERT, NEW YORK, NY, US, Bd. 13, Nr. 2, 1. Februar 1994 (1994-02-01), Seiten 147-152, XP009129907 ISSN: 0272-457X

## Beschreibung

*Clostridium difficile* ist ein obligat anaerobes, Gram-positives Stäbchenbakterium mit einer Länge von 3-6 µm und einer Breite von ca. 0,5 µm. Die Gattung *Clostridium* gehört zur Familie der *Bacillaceae.* Die Entdeckung von *C. difficile* als ein wichtiger Erreger der Antibiotika-assoziierten Kolitis erfolgte Ende der 70er Jahre. Der Name C. *difficile* geht auf das langsame Wachstum in der Kultur und die schwierige Isolierung des Bakteriums zurück. Auf supplementierten Blutagar-Platten wachsen große, grau durchscheinende Kolonien ohne Hämolyse. Wie alle Clostridien besitzt *C. difficile* die Fähigkeit zur Sporenbildung. Auf diese Weise können die Bakterien auch unter extremen Umweltbedingungen überleben

Gesunde Erwachsene tragen zu 2-7% *C. difficile* im Gastrointestinaltrakt. Während einer Antibiotika-Therapie wird der protektive Effekt der physiologischen Flora gestört und es kann im Darm zu einer Überwucherung mit C. *difficile* kommen. Die daraus resultierende Clostridium-difficile-assoziierte Erkrankung (CDAD) variiert hinsichtlich ihres Schweregrades in Abhängigkeit von der Virulenz des vorliegenden C. *difficile* Stamms. Das klinische Bild reicht von leichter Diarrhö bis zur schweren pseudomembranösen Kolitis mit Fieber und krampfartigen Bauchschmerzen und der Gefahr von schweren Komplikationen wie Kolonperforation, Sepsis und toxisches Megakolon. Als Virulenzfaktoren gelten in erster Linie ein Enterotoxin (Toxin A) und ein Zytotoxin (Toxin B). Die Toxine gehören zur Familie der großen clostridialen Toxine (LCT, Large clostridial toxins) und besitzen eine Glykosyltransferase-Aktivität. Darüber hinaus besitzen einige Stämme die Gene für das binäre Toxin CDT (ADP-Ribosyltransferase), das als weiterer Virulenzfaktor diskutiert wird.

Bei den *C*. *difficile* Stämmen unterscheidet man zwischen den sogenannten Standard-Stämmen und deren Varianten. Als Standard *C*. *difficile* Stämme werden in der Regel Stämme vom Toxinotyp 0 bezeichnet. Dazu zählen Stämme mit unterschiedlichen Ribotypen, z.B. 001, 003 und 012. Stämme vom Toxinotyp 0 sind gekennzeichnet durch das Vorhandensein der Gene für die beiden Toxine A und B, dagegen fehlen die Gene für das binäre Toxin.

In den letzten Jahre zeigte sich, dass es neben den Standard *C*. *difficile* Stämmen auch eine Vielzahl unterschiedlicher varianter *C*. *difficile* Stämme gibt, die zunehmend klinische Signifikanz besitzen und auch in Haustieren (insbesondere Hunden, Katzen) und Nutztieren (insbesondere Kälbern und Schweinen) nachgewiesen wurden.

So wurden seit 2003 in Kanada, den USA, Großbritannien und vielen anderen Ländern Europas endemische, häufig schwer verlaufende Infektionen mit varianten *C. difficile* Stämmen beobachtet.

Die im Zusammenhang mit diesen schwer verlaufenden Infektionen isolierten varianten *C. difficile* Stämme besitzen sowohl die Gene für die Toxine A (*tcdA*) und B (*tcdB*) als auch die Gene für das CDT und weisen in ihrem Regulatorgen *tcdC* partielle Deletionen auf.

Diese hoch virulenten *C. difficile* Stämme vom PCR-Ribotyp 027, Toxinotyp III, REA-B1 und PFGE NAP1 besitzen im Regulatorgen *tcdC* eine 18 bp Deletion und einen Frameshift an Position 117, was als Ursache für ihre in vitro nachgewiesene gesteigerte Toxinproduktion und als ein Grund für ihre erhöhte Virulenz vermutet wird. Diese Stämme sind desweiteren dadurch gekennzeichnet, dass sie im Vergleich zu den übrigen *C. difficile* Stämmen veränderte Oberflächenproteine und eine erhöhte Adherenz zu menschlichen Darm-Epithelzellen aufweisen. Außerdem sind diese Stämme resistent gegenüber Antibiotika der Wirkstoffklasse Fluoroquinolone und gegenüber dem Antibiotikum Erythromycin - mutmaßlich aufgrund von Mutationen in den Genen für gyrA/B und die 23sRNA.

Im folgenden werden diese hoch virulenten *C. difficile* Stämme mit dem Kürzel *"C.d.-027-hv"* benannt.

Waren bisher von einer Infektion mit *C. difficile* meist Patienten über 60 Jahre betroffen, wird C.d.-027-hv auch bei jüngeren Patienten und auch außerhalb von Krankenhäusern bei Patienten mit bislang geringem Risiko nachgewiesen. Seine Letalität ist gegenüber den bisher nachgewiesenen *C*. *difficile-* Stämmen um das Fünffache erhöht.

Eine andere Gruppe klinisch relevanter, varianter *C*. *difficile* Stämme wird als Ribotyp 017, Toxinotyp VIII und Serotyp F klassifiziert. Diese Stämme sind wesentlich dadurch gekennzeichnet, dass sie eine 1,7kB große Deletion im *tcdA* Gen aufweisen und aufgrund eines vorzeitigen Stop-Codons kein Toxin A exprimieren. Die Stämme weisen eine Antibiotika-Resistenz gegen Clindamycin und Fluoroquinolone auf, es fehlen die Gene für das binäre Toxin CDT, und zudem weist das Regulatorgen *tcdC* keine Deletion auf. Ein gehäuftes Auftreten solcher Stämme wird u.a. aus den USA, Japan, Israel, Irland und den Niederlanden berichtet. Die Mortalität bei Patienten mit solchen Infektionen liegt mit 14-66% über derjenigen der Standardstämme.

Im folgenden werden diese hoch virulenten *C. difficile* Stämme mit dem Kürzel *"C.d.-017-hv"* benannt.

Der diagnostische Nachweis von *C*. *difficile* erfolgt üblicherweise über den Nachweis von Toxin A, in manchen Fällen auch über den kombinierten Nachweis von Toxin A und B. Ein Nachweis varianter *C*. *difficile* Stämme ist mit diesen Verfahren nicht möglich. Die varianten Stämme werden entweder gar nicht (Toxin A negative Stämme) oder nicht separat detektiert.

Zur Identifizierung und Charakterisierung varianter *C*. *difficile* Stämme ist es notwendig, diese zu typisieren. Verfahren zur Typisierung von *C*. *difficile* sind im Stand der Technik bekannt, z.B. die Toxinotypisierung, die Serotypisierung, die Ribotypisierung oder die Typisierungen mittels PFGE oder REA. Alle diese Verfahren sind jedoch arbeits- und zeitintensiv, sind nur von spezialisierten Laboratorien durchzuführen und erfordern vorab die aufwändige Isolierung der fraglichen *C*. *difficile* Stämme.

Zur genauen Identifizierung spezifischer *C*. *difficile* Stämme werden darüber hinaus meist noch weitere Untersuchungen gefordert, wie z.B. im Fall von *C.d.-027-hv* der Nachweis der Antibiotika-Resistenz und der Deletion im *tcdC* Gen.

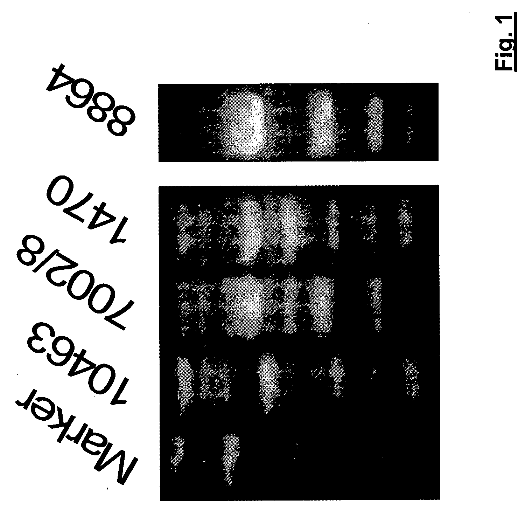

In der Laborpraxis wird deshalb z.B. bei einer Untersuchung hinsichtlich einer C.d.-027-hv-Präsenz wie folgt verfahren:
1. Nachweis von *C*. *difficile* allgemein durch Nachweis der Toxine A und/oder B, im Stuhl der Erkrankten mittels Immunoassay,
2. wenn positiv: Anzucht der *C*. *difficile* Bakterien aus dem Untersuchungsmaterial,
3. bei erfolgreicher Isolierung der Bakterien: Testung auf das Vorliegen der Erythromycin und Moxifloxacin-Resistenz im E-Test,
4. bei positivem Resistenznachweis: Testung auf das Vorliegen des Ribotyps 027 mittels DNA-Präparation und PCR Amplifikation der ribosomalen RNA-Gene (Ribotypisierung) [Fig. 1],
5. bei positivem Nachweis des Ribotyps 027: partielle Genotypisierung zum Nachweis der beiden Toxingene *tcdA* und *tcdB,* der Gene *cdtA*/*B* für das binäre Toxin CDT und der Deletionen im Regulatorgen *tcdC,*
6. bei Vorliegen beider getesteten Antibiotikaresistenzen und aller Merkmale des charakteristischen genotypischen Musters: Diagnose eines hypervirulenten *C. difficile* Stamms Ribotyp 027 (*C.d-027-hv*).

Ein Nachteil dieser bisherigen Laborpraxis, die für andere variante und hochvirulente *C. difficile* Stämme ähnlich komplex ist, besteht vor allem darin, dass sie sehr zeitaufwendig ist: Für die Isolierung und Anzucht des Keims, die Bestimmung der Antibiotika-Resistenz und die Ribotypisierung besteht ein Zeitbedarf von 7-10 Tagen.

Für eine optimale Infektionsüberwachung mit dem Ziel, nosokomiale Infektionen (Krankenhausinfektionen) zu verhindern und Infektionsausbrüche zu kontrollieren - beides sowohl zum Schutz der Patienten und des Personals als auch aus Gründen der Kostenersparnis - müssen die Identifikationsverfahren aber vor allem schnell und möglichst genau sein.

Der vorliegenden Erfindung liegt deshalb die Aufgabe zugrunde, ein Verfahren zur schnellen und sicheren Detektion und Identifikation von varianten *C. difficile* Stämmen in einer Probe, insbesondere einer Patientenprobe (eines menschlichen oder tierischen Patienten) bereitzustellen.

Die im Zuge dieser Aufgabe zu lösenden Probleme bestehen insbesondere darin, (i) dass sich die Toxine der verschiedenen *C. difficile* Stämme nicht durch eine einzigartige Region unterscheiden, (ii) dass es nicht trivial ist, spezifische Antikörper gegen die LCTs eines bestimmten *C. difficile* Stammes zu generieren (die EU finanziert z.B. ein spezielles Forschungsprogramm Nr.37870, Acronym "EACCAD", das sich mit der Erforschung der C.d.-027-hv Stämme beschäftigt und unter anderem die Generierung solcher Antikörper gegen die LCTs von C.d.-027-hv zum Ziel hat), (iii) dass das binäre Toxin CDT auch von anderen C. *difficile* Stämmen gebildet wird, und (iv) dass sich Mutationen in bestimmten Genen, wie z.B. tcdC, in der Regel nicht immunolgisch in Stuhlproben nachweisen lassen.

Eine Lösung der Aufgabe besteht in der Bereitstellung eines Verfahrens, dass durch die folgenden Schritte gekennzeichnet ist:
(a) Gewinnung einer Körperausscheidungs- und/oder Körpergewebeprobe eines menschlichen oder tierischen Patienten
(b) In-Kontakt-bringen der Probe mit jeweils wenigstens einem Antikörper aus wenigstens drei der Antikörpergruppen Gruppe I, Gruppe II, Gruppe III, Gruppe IV, Gruppe V und Gruppe VI, wobei
   - Gruppe I: die folgenden Antikörper umfasst: pan-TcdB Antikörper,
   - Gruppe II: die folgenden Antikörper umfasst: Antikörper, die mit den Toxinen TcdB-10463 und TcdB-017 reagieren, nicht hingegen mit den Toxinen TcdB-8864 und TcdB-027
   - Gruppe III: die folgenden Antikörper umfasst: Antikörper, die mit TcdB-10463 reagieren, nicht hingegen mit den Toxinen TcdB-8864, TcdB-017 und TcdB-027
   - Gruppe IV: die folgenden Antikörper umfasst: Antikörper, die mit den Toxinen TcdB-8864, TcdB-017 und TcdB-027 reagieren, nicht hingegen mit dem Toxin TcdB-10463
   - Gruppe V: die folgenden Antikörper umfasst: Antikörper, die mit den Toxinen TcdB-8864 und TcdB-027 reagieren, nicht hingegen mit den Toxinen TcdB-10463 und TcdB-017
   - Gruppe VI: die folgenden Antikörper umfasst: pan-TcdA Antikörper
(c) Detektion der Antikörperreaktion und Erstellen des Reaktionsmusters, wobei jedem in (b) eingesetzten Antikörper jeder Antikörpergruppe I bis VI ein Positiv-Symbol (z.B."+") für eine detektierte positive Antikörperreaktion und ein Negativ-Symbol (z.B."-") für eine detektierte negative Antikörperreaktion zugeordnet wird, und
(d) Vergleich des in (c) gewonnenen Reaktionsmusters mit Referenzmustern, die dadurch gewonnenen werden, dass von den im Stand der Technik bekannten und im Nachweistest auf Präsenz zu überprüfenden C. *difficile* Stämmen Rx₁, Rx₂, Rxᵢ wenigstens eines der bzw. ihrer jeweiligen großen clostridialen Toxine LCT-Rx₁, LCT-Rx₂, LCT-Rxᵢ in einem Reaktionstest mit den in Schritt (b) gewählten Antikörpern aus den wenigstens drei der Antikörper-Gruppen I bis VI eingesetzt wird, und dass die für jedes LCT und die wenigstens drei der Antikörper-Gruppen I bis VI erhaltenen Reaktionsergebnisse derart musterartig detektiert bzw. registriert bzw. abgebildet werden, dass jeder Antikörpergruppe entweder ein Positiv-Symbol (z.B."+") im Fall einer detektierten positive Antikörperreaktion oder ein Negativ-Symbol (z.B. "-") im Fall einer detektierten negative Antikörperreaktion zugeordnet wird, (e) Bewertung einer Übereinstimmung zwischen dem in (c) gewonnenen Reaktionsmusters mit einem Referenzmuster als Indiz dafür, dass in der Probe der *C. difficile* Stamm des betreffenden Referenzmusters vorliegt.

Hierbei und im folgenden bedeuten:
- LCT: Large Clostridial Toxin, nämlich Toxin A und/oder Toxin B von C. *difficile*
- TcdB-10463: Toxin B des *C*. *difficile* Stammes VPI-10463 (= "Standard-Toxin")
- TcdB-8864: Toxin B des *C*. *difficile* Stammes 8864
- TcdB-1470: Toxin B des *C*. *difficile* Stammes 1470
- TcdB-7002/8: Toxin B des *C*. *difficile* Stammes 7002/8
- TcdB-017: Toxin B von *C*. *difficile* Stämmen mit Ribotyp 017
- TcdB-027: Toxin B von *C*. *difficile* Stämmen mit Ribotyp 027
- pan TcdA-Antikörper: Antikörper, der die TcdA-Proteine von allen pathogenen *C. difficile* Stämmen erkennt (z.B. TTC8)
- pan TcdB-Antikörper: Antikörper, der die TcdB-Proteine von allen pathogenen *C. difficile* Stämmen erkennt (z.B. 2CV, TGC35)

Die Lehre des erfindungsgemäßen Verfahrens basiert auf der überraschenden Erkenntnis, dass
(1.) die gegen die LCTs von *C. difficile,* nämlich gegen Toxin A oder Toxin B gerichteten Antikörper in sechs Gruppen unterteilt werden können, die wie folgt charakterisiert sind:
   - Gruppe I =: pan-TcdB Antikörper TGC35 (DSM ACC 2918) und 2CV (DSM ACC 2321) und Antikörper, die mit TGC35 und/oder 2CV funktions- und wirkungsgleich sind;
   - Gruppe II=: Antikörper, die mit dem Standard-Toxin TcdB-10463 und dem varianten TcdB-017 reagieren, nicht aber mit den varianten Toxinen TcdB-8864 und TcdB-027, insbesondere Antikörper TGC23 (DSM ACC2917) und damit funktions- und wirkungsgleiche Antikörper;
   - Gruppe III =: Antikörper, die mit dem Standard-Toxin TcdB-10463 reagieren, nicht aber mit den varianten Toxinen TcdB-8864, TcdB-017 und TcdB-027, insbesondere Antikörper TGC41 (DSM ACC2919) und damit funktions- und wirkungsgleiche Antikörper;
   - Gruppe IV =: Antikörper, die mit den varianten Toxinen TcdB-8864 und TcdB-017 und TcdB-027 reagieren, nicht aber mit dem Standard-Toxin TcdB- 10463, insbesondere Antikörper TGC16 (DSM ACC2915) und damit funktions- und wirkungsgleiche Antikörper;
   - Gruppe V =: Antikörper, die mit den varianten Toxinen TcdB-8864 und TcdB-027 reagieren, nicht aber mit dem Standard-Toxin TcdB-10463 und dem varianten TcdB-017, insbesondere Antikörper TGC19 (DSM ACC2916) und damit funktions- und wirkungsgleiche Antikörper;
   - Gruppe VI =: pan-TcdA Antikörper, die mit den TcdA-Proteinen aller pathogenen *C. difficile* Stämme reagieren, insbesondere Antikörper TTC8 (DSM ACC 2322) und damit funktions- und wirkungsgleiche Antikörper (wie der in US 4879218 beschriebene Antikörper PCG4);
   und
(2.) dass die bisher bekannten virulenten varianten *C. difficile* Stämme bzw. deren LCTs mit den Antikörpern dieser sechs Gruppen - und insbesondere deren Toxin B mit den Antikörpern der fünf Gruppen I bis V - jeweils ein charakteristisches Reaktionsmuster zeigen, welches sich von den Reaktionsmustern der Toxine anderer (hoch-) virulenter varianter *C. difficile* Stämme und von den Reaktionsmustern der Toxine der Standard-*C. difficile* Stämme unterscheidet.

Zum Beispiel ist der hochvirulente variante *C. difficile* Stamm *C.d.-027-hv* durch das Reaktionsmuster "+--++ +" in der Reihenfolge der Gruppen I, II, III, IV, V, VI charakterisiert, während der ebenfalls hochvirulente *C. difficile* Stamm *C.d.-017-hv* durch das Reaktionsmuster "++-+--" (ebenfalls in der Reihenfolge der Gruppen I, II, III, IV, V, VI) gekennzeichnet ist.

Zur Identifikation und Detektion der verschiedenen derzeit bekannten und diagnostisch relevanten virulenten varianten *C*. *difficile* Stämme sind in der Regel jeweils wenigstens ein Antikörper aus mindestens drei verschiedenen Antikörper-Gruppen notwendig und hinreichend. Zum Beispiel ist für die Unterscheidung des *C. difficile* Stammes *C.d.-027-hv* von den übrigen relevanten varianten *C. difficile* Stämmen und von den Standard-*C. difficile* Stämmen das Reaktionsmuster der drei Gruppen II, IV und VI, nämlich "-++" (in der Reihenfolge der Gruppen II, IV, VI) ausreichend, für die Unterscheidung bzw. Identifizierung des *C*. *difficile* Stammes *C.d.-017-hv* ist das Reaktionsmuster der drei Gruppen II, IV und VI, nämlich "+ +-" (ebenfalls in der Reihenfolge der Gruppen II, IV, VI) ausreichend.

Mit dem erfindungsgemäßen Verfahren gehen insbesondere die folgenden Vorteile einher:
Es kann direkt anhand der Stuhlprobe mittels eines einfach durchzuführenden Tests und innerhalb weniger Stunden festgestellt werden, ob ein bestimmter hochvirulenter varianter *C. difficile* Stamm vorliegt oder nicht: Wird z.B. auf die Präsenz von *C.d.-027-hv* untersucht und wird ein Antikörper-Antigen-Reaktionsmuster erhalten, das für einen Antikörper aus wenigstens einer der Antikörpergruppen I, IV, V und/oder VI keine positive Reaktion zeigt, kann zuverlässig diagnostiziert werden, dass kein Stamm vom Typ *C.d.-027-hv* vorliegt. Zeigt das Antikörper-Antigen-Reaktionsmuster hingegen für alle vier Antikörpergruppe I, IV, V und VI eine positive Reaktion, liegt mit sehr hoher Wahrscheinlichkeit eine Infektion mit einem *C.d.-027-hv* Stamm vor. Zur Bestätigung der Verdachtsdiagnose können/sollten dann die zeit- und kostenaufwendigen Spezialanalysen Antibiotikaresistenz, Ribotypisierung, und/oder Genanalyse (insbesondere Nachweis der Toxin-Gene tcdA, tcdB und tcd A/B und des Regulatorgens tcdC mit den charakteristischen Deletionen und Frameshifts) durchgeführt werden.

Mit anderen Worten: Das erfindungsgemäße Verfahren ersetzt in dem herkömmlichen Untersuchungsverfahren die ersten drei Schritte (Toxin-Nachweis, Bakterien-Anzucht, Antibiotika-Resistenztest) und ermöglicht in wesentlich kürzerer Zeit, nämlich wenigen Stunden gegenüber herkömmlicherweise mehreren Tagen, zumindest eine zuverlässige Ausschlussdiagnose in den Fällen, in denen keine Infektion mit dem zu überprüfenden Varianten *C. difficile* Stamm z.B. *C.d.-027-hv,* vorliegt. Die zeit- und kostenaufwendigen weiteren Schritte (4, 5 und 6) im herkömmlichen Untersuchungsverfahren brauchen erst und nur dann durchgeführt werden, wenn das erfindungsgemäße Verfahren auf das Vorhandensein des zu überprüfenden varianten *C*. *difficile* Stammes, z.B. *C.d.-027-hv oder C.d.-017-hv* hinweist.

Auch Vorbeugemaßnahmen wie z.B. Isolierung, Quarantäne, strenge Hygienemaßnahmen können gezielt dann eingesetzt werden, wenn das Risiko einer *C.d.-027-hv*-Infektion oder *C.d.-017-hv*-Infektion aufgrund des Ergebnisses des erfindungsgemäßen Nachweistests tatsächlich hochgradig gegeben ist.

Bei den Antikörpern der Gruppen I bis VI handelt es sich vorzugsweise um monoklonale Antikörper. Grundsätzlich kommen als Antikörper für das erfindungsgemäße Verfahren auch alle dem Fachmann bekannten natürlichen, biotechnologisch hergestellten oder gentechnologisch hergestellten Antikörper-Alternativen in Betracht, z.B. Kamelantikörper (Nanobodies), Affibodies, Anticaline, Cystein-knot Mini-Proteine.

In einer Ausführungsform des Verfahrens, die sich in der Praxis sehr gut bewährt hat, ist der wenigstens eine Antikörper aus Gruppe I der monoklonale Antikörper 2CV (DSM ACC 2321) oder der monoklonale Antikörper TGC 35 (DSM ACC2918), und/oder der wenigstens eine Antikörper aus Gruppe II ist der monoklonale Antikörper TGC23 (DSM ACC2917), und/oder der wenigstens eine Antikörper aus Gruppe III ist der monoklonale Antikörper TGC41 (DSM ACC2919), und/oder der wenigstens eine Antikörper aus Gruppe IV ist der monoklonale Antikörper TGC 16 (DSM ACC2915), und/oder der wenigstens eine Antikörper aus Gruppe V ist der monoklonale Antikörper TGC 19 (DSM ACC2916) und/oder der wenigstens eine Antikörper aus Gruppe VI ist der monoklonale Antikörper TTC8 (DSM ACC 2322).

Bei der Körperausscheidungsprobe handelt es sich in der Praxis vorzugsweise um eine Stuhlprobe, und bei der Körpergewebeprobe handelt es sich vorzugsweise um eine Blutprobe.

Eine weitere Ausführungsform des Verfahrens, die für die Praxis hochrelevant ist, weil sie eine schnelle und.zuverlässige Detektion von varianten *C*. *difficile* Stämmen ermöglicht, ist dadurch gekennzeichnet, dass in Schritt (b) die Probe mit jeweils einem Antikörper aus jeder der Gruppen I, IV und VI in Kontakt gebracht wird, und dass in Schritt (c) eine positive Reaktion der Antikörper aus den Gruppen I, IV und VI das Vorliegen eines hochvirulenten, varianten C. *difficile* Stammes anzeigt.

Eine für die Praxis hoch relevante Ausführungsform des Verfahrens, die eine schnelle und zuverlässige Detektion des hochvirulenten *C*. *difficile* Stammes Ribotyp 027, PFGE-NAP1, REA-B1 und Toxinotyp III ermöglicht und damit die Überprüfung von C.d.-027-hv Verdachtsfällen, ist dadurch gekennzeichnet, dass in Schritt (b) die Probe mit jeweils wenigstens einem Antikörper aus jeder der Antikörpergruppen Gruppe II, Gruppe IV und Gruppe VI in Kontakt gebracht wird, und dass in Schritt (c) eine positive Reaktion der Antikörper aus Gruppe IV und Gruppe VI und eine negative Reaktion des/der Antikörper(s) aus Gruppe II das Vorliegen eines hochvirulenten varianten *C*. *difficile* Stammes vom Typ Ribotyps 027, PFGE-NAP1, REA-B1, Toxinotyp III anzeigt.

Diese Ausführungsform kann noch erweitert werden, indem in Schritt (b) die Probe zusätzlich mit jeweils wenigstens einem Antikörper der Antikörpergruppe I und/oder der Antikörpergruppe III und/oder der Antikörpergruppe V in Kontakt gebracht wird, und indem in Schritt (c) zusätzlich eine positive Reaktion des/der Antikörper(s) aus Gruppe I und Gruppe V und eine negative Reaktion des/der Antikörper(s) aus Gruppe III das Vorliegen eines hochvirulenten varianten *C*. *difficile* Stammes vom Typ Ribotyps 027, PFGE-NAP1, REA-B1, Toxinotyp III anzeigt.

Eine weitere, für die Praxis ebenfalls hoch relevante Ausführungsform des Verfahrens, die eine schnelle und zuverlässige Detektion des hochvirulenten *C. difficile* Stammes Ribotyp 017, Toxinotyp VIII und Serotyp F ermöglicht und damit die Überprüfung von *C.d.-017-hv* Verdachtsfällen, ist dadurch gekennzeichnet, dass in Schritt (b) die Probe mit jeweils wenigstens einem Antikörper aus jeder der Antikörpergruppen Gruppe II, Gruppe IV und Gruppe VI in Kontakt gebracht wird, und dass in Schritt (c) eine positive Reaktion der Antikörper aus Gruppe II und Gruppe IV und eine negative Reaktion des/der Antikörper(s) aus Gruppe VI das Vorliegen eines hochvirulenten varianten C. *difficile* Stammes vom Typ Ribotyps 017, Toxinotyp VIII und Serotyp F anzeigt.

Diese Ausführungsform kann noch erweitert werden, indem in Schritt (b) die Probe zusätzlich mit jeweils wenigstens einem Antikörper der Antikörpergruppe I und/oder der Antikörpergruppe III und/oder der Antikörpergruppe V in Kontakt gebracht wird, und indem in Schritt (c) zusätzlich eine positive Reaktion des/der Antikörper(s) aus Gruppe I und eine negative Reaktion der Antikörper aus Gruppe III und Gruppe V das Vorliegen eines hochvirulenten varianten *C*. *difficile* Stammes vom Typ Ribotyps 017, Toxinotyp VIII und Serotyp F anzeigt.

Die der vorliegenden Erfindung zugrunde liegende Aufgabe wird auch mit der Bereitstellung des monoklonalen Antikörpers gelöst, der von der bei der DSMZ, Braunschweig, Deutschland, seit dem 05. Juni 2008 unter der Nummer DSM ACC2916 (TGC 19) hinterlegten Hybridomazellinie produziert wird, und der zur Verwendung in dem erfindungsgemäßen Verfahren zum Nachweis und zur Identifikation von varianten *C. difficile* Stämmen, insbesondere in der Verfahrensvariante zum Nachweis und zur Identifikation des *C*. *difficile* Stammes Ribotyp 027, PFGE-NAP1, REA-B1 und Toxinotyp III geeignet und vorgesehen ist.

Die genannte Aufgabe wird ferner mit der Bereitstellung des monoklonalen Antikörpers gelöst, der von der bei der DSMZ, Braunschweig, Deutschland seit dem 05. Juni 2008 unter der Nummer DSM ACC2917 (TGC 23) hinterlegten Hybridomazellinie produziert wird, und der zur Verwendung in dem erfindungsgemäßen Verfahren zum Nachweis und zur Identifikation von varianten *C*. *difficile* Stämmen, insbesondere in der Verfahrensvariante zum Nachweis und zur Identifikation des *C*. *difficile* Stammes Ribotyp 017, Toxinotyp VIII und Serotyp F geeignet und vorgesehen ist.

Die genannte Aufgabe wird außerdem mit der Bereitstellung des monoklonalen Antikörpers gelöst, der von der bei der DSMZ, Braunschweig, Deutschland unter der Nummer DSM ACC2915 (TGC 16) hinterlegten Hybridomazellinie produziert wird, und der zur Verwendung in dem Verfahren zum Nachweis und zur Identifikation eines varianten *C. difficile* Stammes in einer Probe, insbesondere, insbesondere in der Verfahrensvarianten zum Nachweis und zur Identifikation von *C. difficile* Stämmen mit dem Ribotyp 017, Toxinotyp VIII und Serotyp F und/oder von *C. difficile* Stämmen mit dem Ribotyp 027, PFGE-NAP1, REA-B1 und Toxinotyp III geeignet und vorgesehen ist.

Die genannte Aufgabe wird schließlich auch durch die Bereitstellung des monoklonalen Antikörpers gelöst, der von der bei der DSMZ, Braunschweig, Deutschland unter der Nummer DSM ACC2918 (TGC 35) hinterlegten Hybridomazellinie produziert wird, und der zur Verwendung in dem Verfahren zum Nachweis und zur Identifikation eines varianten *C. difficile* Stammes in einer Probe, insbesondere, insbesondere in der Verfahrensvarianten zum Nachweis und zur Identifikation von *C. difficile* Stämmen mit dem Ribotyp 017, Toxinotyp VIII und Serotyp F und/oder von *C. difficile* Stämmen mit dem Ribotyp 027, PFGE-NAP1, REA-B1 und Toxinotyp III gecignet und vorgesehen ist

Die Erfindung wird im folgenden anhand von Ausführungsbeispielen näher erläutert:
Die in den Beispielen genannten Figuren zeigen:
   - **Fig. 1:**: **die Ribotypisierung der verwendeten Stämme.** Die Stämme wurden nach den Angaben von Stubbs et al. ribotypisiert und über Abgleich mit Referenzstämmen den unterschiedlichen Ribotypen zugeordnet. Dabei wurde Stamm VPI-10463 und Stamm 8864 bislang unbekannten Ribolypen und Stamm 1470 Ribotyp 017 und Stamm 7002/8 Ribotyp 027 zugeordnet. Die Marker-Banden laufen bei 600bp, 500bp, 400bp und 300bp (von oben).

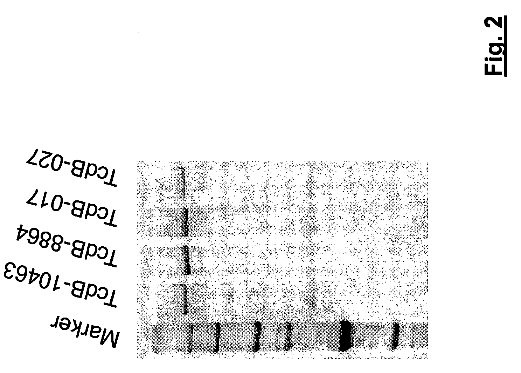

   - **Fig. 2:**: **gereinigte Toxin B Varianten.** Verschiedene Toxin B Varianten sind im Coomasi-gefärbten SDS-PAGE dargestellt. Von den einzelnen Toxinen wurden zwischen 250-400ng pro Spur aufgetragen

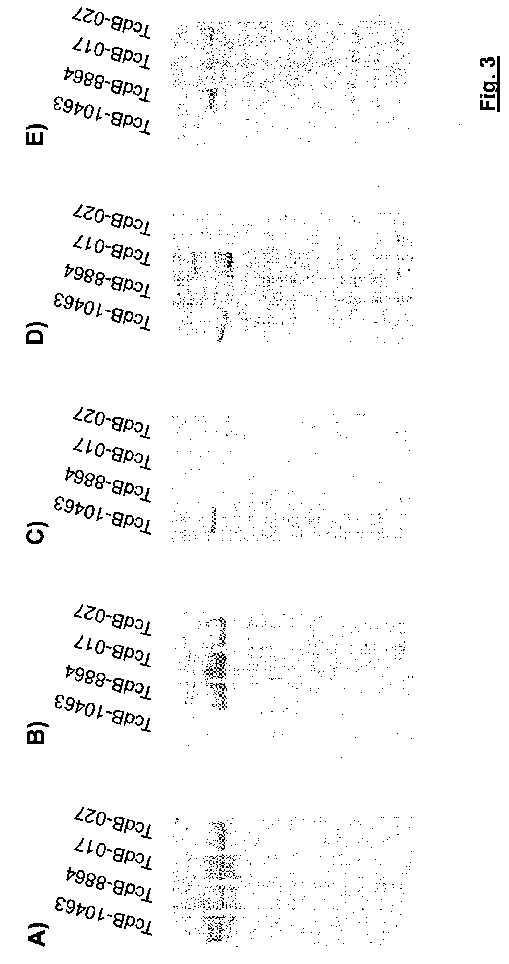

   - **Fig. 3:**: **den Nachweis der Spezifität monoklonaler anti-Toxin B Antikörper.** Verschiedene Toxin B Varianten wurden im SDS-PAGE aufgetrennt, auf Nylonmembranen geblotet und anschließend mit den entsprechenden Antikörpern entwickelt, Gezeigt sind die Spezifitäten der monoklonalen Antikörper TGC35 (Fig. 3 **A**), TGC16 (Fig. 3 **B**), TGC41 (Fig. 3 **C**), TGC23 (Fig. 3 **D**) und TGC19 (Fig. 3 **E**).

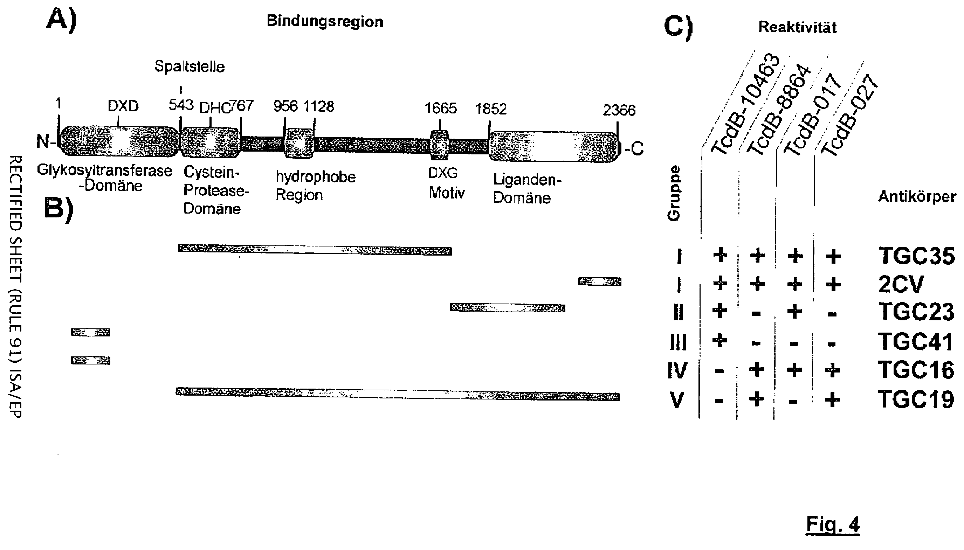

   - **Fig. 4:**: **die Bindungsregionen der monoklonalen Antikörper.** Fig. 4 **A** ist eine schematische Darstellung der Struktur des Toxin B. Fig. 4 **B** stellt die Bindungsregion der Antikörper innerhalb des Toxin B dar. Fig. 4C zeigt die Reaktivitäten der monoklonalen Antikörper.

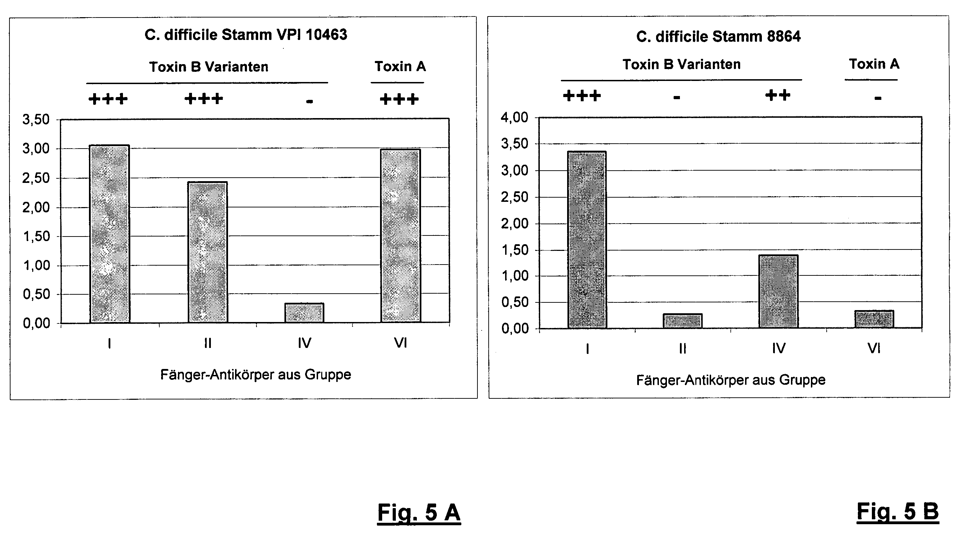

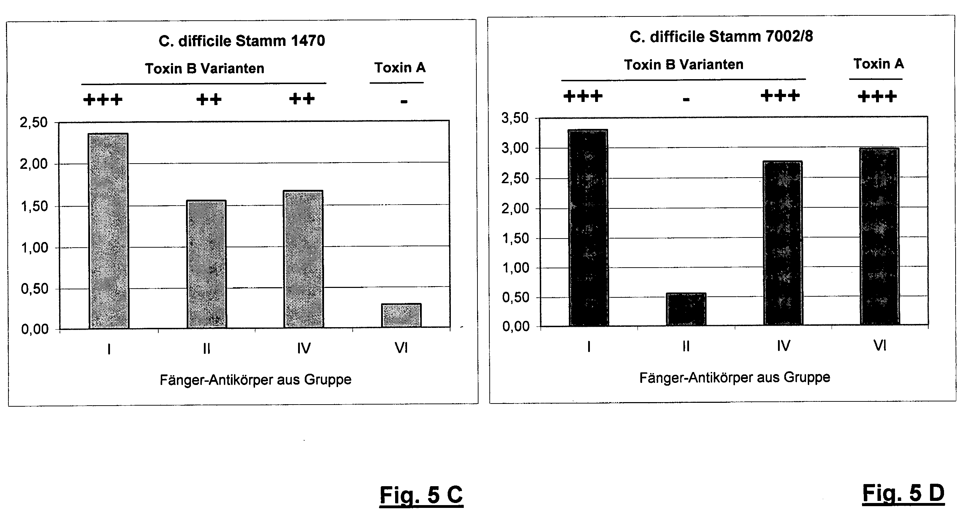

   - **Fig. 5:**: **Referenzmuster von C. difficile Stämmen.** Die Toxin A und Toxin B Expression im Kulturüberstand von vier verschiedenen C. difficile Stämme wurde mittels Sandwich-ELISA unter Einsatz von Fänger-Antikörper aus den Antikörper-Gruppen I, II, IV und VI getestet. Als Fänger-Antikörper wurden eingesetzt aus Gruppe I: TGC35, aus Gruppe II: TGC23, aus Gruppe IV: TGC16, Gruppe VI: TTC8. Gezeigt sind die Erkennungsmuster der C. difficile Stämme VPI-10463 (Fig. 5 **A**), 8864 (Fig. 5 **B**), 1470 Ribotyp 017 (Fig. 5 **C**) und 7002/8 Ribotyp 027 (Fig. 5 **D**)

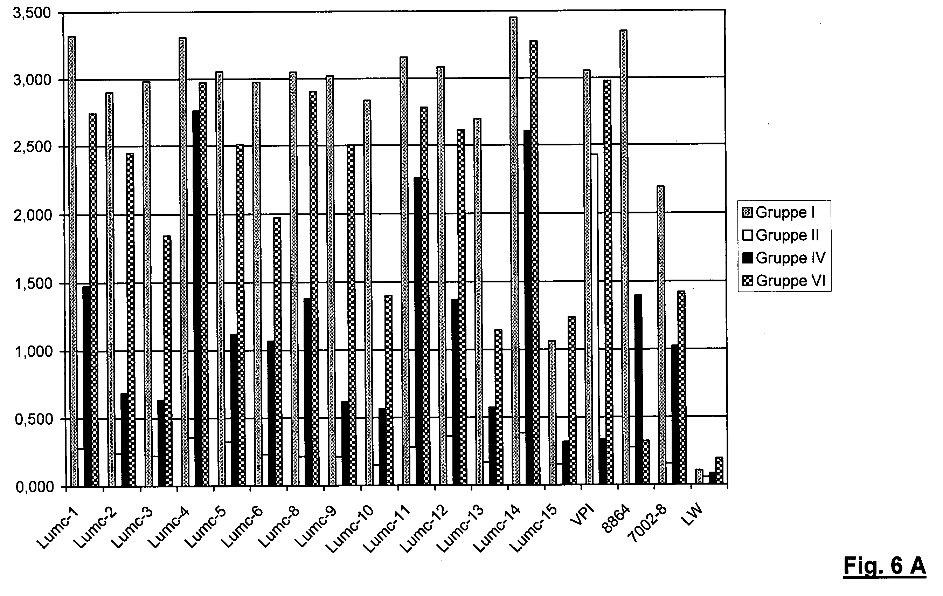

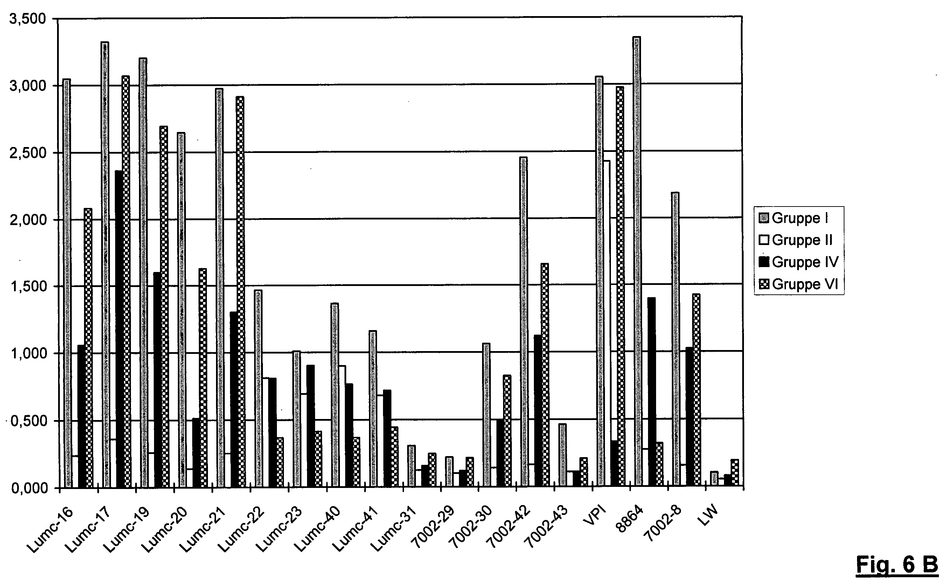

   - **Fig. 6:**: **Reaktionsmuster verschiedener *C*. *difficile* Stämme im Fänger-ELISA.** Als Identifizierungsantikörper wurde aus Gruppe I der TGC35, aus Gruppe II der TGC23, aus Gruppe IV der TGC16 und aus Gruppe VI der TTC8 verwendet. In Fig. 6A sind die Ergebnisse von 17 verschiedenen *C*. *difficile* Stämmen und in Fig. 6B sind die Ergebnisse von weiteren *C. difficile* Stämmen abgebildet.

### Beispiel 1: Herstellung von monoklonalen Antikörpern, die spezifisch mit Toxin B von C. difficile reagieren

### (A) Gewinnung von gereinigtem C. difficile Toxin B aus verschiedenen C. difficile Stämmen

Verwendet wurden die C. *difficile* Stämme:
VPI-10463 (ATCC43255),
8864 (CCUG20309),
1470 (ATCC43598), und
7002/8 (humanes Isolat aus einer Patientenprobe).

Die Stämme wurden gemäß dem Ribotypisierungsverfahren nach Stubbs et al. 1999 ribotypisiert. Die dabei erhaltenen Ergebnisse sind in Fig. Abb. 1 dargestellt. Es zeigte sich, dass Stamm 1470 dem Ribotyp 017 und Stamm 7002/8 Ribotyp 027 entspricht, während die Stämme VPI-10463 und 8864 bisher noch nicht offiziell benannten Ribotypen zuzuordnen sind.

Das von Stamm VPI-10463 exprimierte Toxin B (TcdB-10463) wird im Stand der Technik generell als Standard-Toxin bezeichnet.

Stamm 8864 produziert eine Variante dieses Toxin B (TcdB-8864), die sich vor allem in der C-terminalen Rezeptorbindungs-Domaine von dem Standard-Toxin unterscheidet (Soehn et al. 1998).

Stamm 1470 exprimiert ein Toxin B (TcdB-1470), das sich insbesondere in der N-terminalen enzymatischen Domaine von dem Standard-Toxin B unterscheidet (von Eichel-Streiber et al. 1995).

Die Sequenz des Toxin B des Stammes 7002/8 Ribotyp 027 ist nicht bekannt. Dagegen wurden die Sequenzen des TcdB-027 in zwei anderen *C.d.-027-hv* Stämmen bestimmt (Stamm QCD-32g58, Genbank-Acc. Nr. NZ_AML00000000, Stamm R20291, www.sanger.ac.uk). Ein Vergleich dieser TcdB-027 Sequenzen mit dem Standard-Toxin B und anderen varianten TcdB-Toxinen ergibt, dass TcdB-027 in der N-terminalen, enzymatischen Domäne dem Standard-Toxin TcdB-10463 ähnelt, in der C-terminalen Domäne jedoch dem varianten Toxin TcdB-8864.

Zur Reinigung der Toxine wurden die Bakterien in Brain Heart Infusion Nährmedien (Becton Dickinson, Heidelberg, Deutschland) anaerob angezogen. Anschließend wurden die Toxine A und B aus dem Überstand dieser Kulturen gereinigt, wie in Moos et al. (2000) beschrieben. Die Reinheit der Toxine wurde im Coomassie-Gel überprüft (vgl. Fig. 2).

Das isolierte und aufgereinigte TcdB der *C. difficile* Stämme VPI-10463, 8864 und 1470 wurden mit den im Stand der Technik bekannten und geläufigen Verfahren inaktiviert und der Erfolg der Inaktivierung im Zytotoxizitätstest überprüft.

### (B) Gewinnung von monoklonalen Antikörpern gegen die Toxin-B-Varianten aus (A)

In einer Reihe von Ansätzen wurden jeweils mehrere Mäuse mit den inaktivierten Toxin B Varianten TcdB-8864 und TcdB-1470 und dem Standard-Toxin TcdB 10163 zunächst grundimmunisiert und nachfolgend zwei Boosterimmunisierungen unterworfen.

Der Titer der Tiere gegen das Immunogen wurde bestimmt und die Milz entnommen, falls sich ein signifikanter Titer entwickelt hatte. Die isolierten Milzzellen wurden mit den im Stand der Technik bekannten und geläufigen Verfahren mit der Maus-Myelomzelllinie X63Ag8.653 (ATCC CRL1580) fusioniert und die Fusionszellen anschließend selektioniert und kloniert. Die dabei gewonnenen Hybridoma-Klone wurden immunologisch auf die Produktion spezifischer monoklonaler Antikörper gegen das zur Immunisierung verwendete Toxin B gescreent.

Ingesamt wurden 15 Fusionen durchgeführt und schließlich 21 verschiedene Hybridomazelllinien isoliert, die Toxin B spezifische Antikörper exprimieren.

Für die weiteren Untersuchungen wurden die Antikörper aus Zellkultur-Überstand gereinigt. Hierfür wurden die Hybridoma-Zelllinien in serumfreiem Medium ISF-1 (Biochrom, Berlin, Deutschland) angezogen und anschließend in einem Volumen von 250ml in einem Spinner-System (Bellco, Vineland, USA) bei 50rpm kultiviert, bis die Vitalität der Kultur auf weniger als 30% abfiel. Der Überstand der Kultur wurde mittels einer Protein A Säule (HiTrap MabSelect SuRe, GE Healthcare, Freiburg, Deutschland) aufgereinigt. Die Konzentration der gereinigten Antikörper lag bei 1-2mg/ml, die Reinheit wurde im Coomassie-Gel überprüft.

### Beispiel 2: Charakterisierung der Anti-Toxin B-Antikörper

Zur Charakterisierung der in Beispiel 1 generierten Antikörper wurden diese im Western Blot getestet.

Hierzu wurden zunächst jeweils 250-400ng des Standard Toxin-B-10463 und der Toxin B Varianten TcdB-8864, TcdB-017 und TcdB-027 im SDS-PAGE aufgetrennt und anschließend im Semidry-Blotverfahren auf Nylonmembranen geblotet. Die Entwicklung erfolgte nach den im Stand der Technik bekannten Verfahren.

Zur Detektion der Toxine wurden die in Beispiel 1 generierten monoklonalen Antikörper eingesetzt.

Die Ergebnisse für die Antikörper mit der Laborbezeichnung TGC 16, TGC 19, TGC 23, TGC 35 und TGC 41 sind in Fig. 3 zusammengefasst:
Die Ergebnisse zeigen, dass die meisten generierten Antikörper eine Untergruppe verschiedener Toxin B Varianten erkennen und nicht monospezifisch nur eine bestimmte Toxin-Variante.

Es wurden insgesamt 11 verschiedene Antikörper identifiziert, die an das Standard-Toxin B und an alle Toxin B Varianten binden. Ein charakteristischer Vertreter dieser sogenannten pan-TcdB Antikörper ist der Antikörper TGC 35. Die Hybridoma Zelllinie, die diesen TGC 35 produziert, ist bei der DSMZ (Deutsche Sammlung für Mikroorganismen und Zellkulturen, Braunschweig, Deutschland) seit dem 05. Juni 2008 unter der Nummer DSM ACC2918 hinterlegt.

Einige Antikörper reagieren sowohl mit dem Standard-Toxin als auch mit dem Toxin TcdB-017. Ein charakteristischer Vertreter dieser Antikörper-Gruppe ist Antikörper TGC 23. Die Hybridoma Zelllinie, die diesen TGC 23 produziert, ist bei der DSMZ seit dem 05. Juni 2008 unter der Nummer DSM ACC2917 hinterlegt

Einige Antikörper reagieren spezifisch mit dem Standard-Toxin TcdB-10463. Ein charakteristischer Vertreter dieser Antikörper-Gruppe ist Antikörper TGC 41. Die Hybridoma Zelllinie, die diesen TGC 41 produziert, ist bei der DSMZ seit dem 05. Juni 2008 unter der Nummer DSM ACC2919 hinterlegt.

Überraschenderweise konnten auch zwei Antikörper generiert werden, die mit einer oder mehreren der Toxin B-Varianten, jedoch nicht mit dem Standard-Toxin B reagieren.

Der Antikörper TGC 16 bindet nicht an das Standard-Toxin B, wohl aber an die varianten Toxine TcdB-8864, TcdB-017 und TcdB-027. Die Hybridoma Zelllinie, die diesen TGC 16 produziert, ist bei der DSMZ seit dem 05. Juni 2008 unter der Nummer DSM ACC2915 hinterlegt.

Der Antikörper TGC 19 bindet nicht an das Standard-Toxin B oder das variante Toxin TcdB-017, wohl aber an die varianten Toxine TcdB-8864 und TcdB-027. Die Hybridoma Zelllinie, die diesen TGC 19 produziert, ist bei der DSMZ seit dem 05. Juni 2008 unter der Nummer DSM ACC2916 hinterlegt.

Die Antikörper wurden in einem Fänger-ELISA hinsichtlich ihrer Antigen-Nachweisgrenze untersucht. Hierfür wurden ELISA-Platten pro well mit 50µl Coatingbuffer (50mM Na₂CO₃, 50mM NaHCO₃, pH 9,6) und 250ng des jeweiligen Antikörpers für 3h bei Raumtemperatur inkubiert und dabei der Antikörper an die ELISA-Platte angelagert. Unspezifische Bindungsstellen wurden durch nachfolgende Inkubation mit 200µl 5x WAPU (12,5ml TritonX100, 62,5ml 1M Tris-HCl pH 7,5, 62,5ml einer 20%igen Gelatinelösung, ad 500ml mit H₂O) für die Dauer von 30 Minuten blockiert. Anschließend wurden unterschiedliche Konzentrationen der Antigene, nämlich im Fall von TGC16 und TGC 19 des Antigens TcdB-027 und im Fall von TGC 23 und TGC 35 des Antigens TcdB-017 appliziert. Die Detektion erfolgte mit polyklonalem Serum (266ng/ml). Die Reaktionsergebnisse (hier nicht dargestellt) zeigen, dass für die Antikörper TGC 16 und TGC 23 die Detektionsgrenze für das jeweilige Toxin B bei <300pg/ml liegt, für Antikörper TGC 35 liegt sie bei unter 1ng/ml und für Antikörper TGC 19 liegt sie bei 1ng/ml.

Die Antikörper TGC 16, TGC 19, TGC 23, TGC 35 und TGC 41 wurden hinsichtlich ihrer Bindungsorte am Toxin B untersucht. Durch proteolytischen Verdau des Toxin B oder durch die Klonierung und Expression rekombinanter Toxin B Teilfragmente in E. coli wurden Proteinfragmente des Toxin B hergestellt. Diese Toxin B Teilfragmente wurden in Western Blot Experimenten aufgetrennt und die Blots anschließend mit den Antikörpern TGC 16, TGC 19, TGC 23, TGC 35 und TGC 41 inkubiert. Die Ergebnisse dieser Versuche sind in Fig. 4 schematisch zusammengefasst und zeigen, dass die einzelnen Antikörper unterschiedliche Regionen des Toxin B Moleküls erkennen bzw. binden:
Alle Aminosäurepositionsangaben sind in Bezug auf die entsprechenden Positionen im Standard-Toxin TcdB-10463 angegeben.

TGC 16 bindet TcdB-8864, TcdB-017 und Tcd-027 jeweils innerhalb der Glykosyltranferasedomaine und dort im Bereich der Aminosäurepositionen 122 bis 261. TGC 19 bindet TcdB-8864 und Tcd-027 jeweils in dem Bereich jenseits der Glykosyltranferasedomaine zwischen den Aminosäurepositionen 543 und 2366.

TGC 23 bindet Standard-Toxin und TcdB-017 jeweils in der Ligandendomaine mit Abstand vom C-terminalen Ende zwischen den Aminosäurepositionen 1692 und 2113. TGC 35 bindet Standard-Toxin, TcdB-8864, TcdB-017 und Tcd-027 jeweils innerhalb der Translokationsdomaine zwischen der Glykosyltranferasedomaine am N-terminalen Ende des Toxins und der Ligandendomaine am C-terminalen Ende zwischen den Aminosäurepositionen 543 und 1692 .

TGC 41 bindet Standard-Toxin analog des TGC 16, das heißt innerhalb der Glykosyltranferasedomaine und dort im Bereich der Aminosäurepositionen 122 bis 261. TGC 16 und TGC 41 binden in der gleichen Toxin-B-Region, nämlich in der Glykosyltranferasedomaine, jedoch von verschiedenen Toxin-B-Varianten, nämlich von Standard-Toxin einerseits (TGC 41) und von TcdB-8864, TcdB-017 und Tcd-027 andererseits (TGC 16). Damit ist erstmals eine Region im Toxin-B identifiziert worden, die eine spezifische Unterscheidung zwischen dem Standard-Toxin-B TcdB-10463 und den varianten Toxinen TcdB-8864, TcdB-017 und Tcd-027 ermöglicht. Überraschenderweise handelt es sich bei dieser Region gerade um die Glykosyltranferasedomaine, die die enzymatische Aktivität des Toxins B vermittelt. Die enzymatische Aktivität ist grundsätzlich bei allen Toxin B dieselbe.

Mit den im Zuge der vorliegenden Erfindung neu generierten Antikörpern lassen sich verschiedene Gruppen (Klassen) von Antikörpern (Antikörpergruppen/Antikörperklassen) aufstellen, die sich hinsichtlich ihrer Reaktivität mit dem Standard-Toxin B und mit den verschiedenen Toxin B-Varianten voneinander unterscheiden:
Die Gruppe der pan-TcdB-Antikörper wird im folgenden als Gruppe I bezeichnet. Typische Vertreter dieser Gruppe sind die monoklonalen Antikörper TGC 35 und 2CV (bekannt aus EP 0994904).

Die Gruppe derjenigen Antikörper, die sowohl mit dem Standard-Toxin als auch mit dem Toxin TcdB-017 reagiert, jedoch nicht mit den varianten Toxinen TcdB-027 und TcdB-8864 wird im folgenden als Gruppe II bezeichnet. Ein typischer Vertreter dieser Gruppe ist der monoklonale Antikörper TGC 23.

Die Gruppe derjenigen Antikörper, die mit dem Standard-Toxin TcdB-10463 reagiert, nicht aber mit den varianten Toxinen TcdB-027, TcdB-8864 und TcdB-1470, wird im folgenden als Gruppe III bezeichnet. Ein typischer Vertreter dieser Gruppe ist der monoklonale Antikörper TGC 41.

Die Gruppe derjenigen Antikörper, die nicht mit dem Standard-Toxin B, wohl aber mit den Toxinen TcdB-8864, TcdB-017 und TcdB-027 interagiert, wird im folgenden als Gruppe IV bezeichnet. Ein typischer Vertreter dieser Gruppe ist der monoklonale Antikörper TGC 16.

Die Gruppe derjenigen Antikörper, die nicht mit dem Standard-Toxin B oder dem Toxin TcdB-017, wohl aber mit den Toxinen TcdB-8864 und TcdB-027 reagiert, wird im folgenden als Gruppe V bezeichnet. Ein typischer Vertreter dieser Gruppe ist der monoklonale Antikörper TGC 19.

Aus der Literatur ist außerdem die Gruppe der pan-TcdA-spezifischen Antikörper bekannt, die im folgenden als Gruppe VI bezeichnet wird. Ein typischer Vertreter dieser Gruppe ist der monoklonale Antikörper TTC8 (bekannt aus EP 0994904).

### Beispiel 3: Detektion von LCTs im Fänger-ELISA - Erstellung von Antigen-Antikörper-Referenzmustern für bekannte variante C. difficile Stämme unter Verwendung der Antikörper-Gruppen I, II, IV und VI

Grundsätzlich wird die Erstellung von Antigen-Antikörper-Referenzmustern folgendermaßen durchgeführt: Von im Stand der Technik bekannten und im Nachweistest auf Präsenz zu überprüfenden *C. difficile* Stämmen Rx₁, Rx₂, Rxᵢ wird jeweils wenigstens das Toxin B, vorzugsweise beide großen clostridialen Toxine (LCTs) gewonnen (in Form des Kulturüberstandes oder der gereinigten Toxine). Mit diesen bekannten Toxinen LCT-Rx₁, LCT-Rx₂, LCT-Rxᵢ wird ein Reaktionstest mit jeweils wenigstens einem Antikörper aus vorzugsweise jeder der Antikörper-Gruppen I bis VI durchgeführt. Die für jedes LCT (LCT-Rx₁, LCT-Rx₂, LCT-Rxᵢ) und die eingesetzten Antikörper-Gruppen erhaltenen Reaktionsergebnisse werden derart musterartig registriert, dass jeder Antikörpergruppe entweder ein Positiv-Symbol (z.B."+") im Fall einer detektierten positiven Antikörperreaktion oder ein Negativ-Symbol (z.B. "-") im Fall einer detektierten negativen Antikörperreaktion zugeordnet wird.

Im vorgestellten Beispiel wurde konkret wie folgt verfahren:
ELISA-Platten wurden mit den in Beispiel 2 beschriebenen gereinigten Antikörpern TGC35 (Gruppe I), TGC23 (Gruppe II), TGC16 (Gruppe IV) und TTC8 (Gruppe VI) wie folgt bestückt: Pro well wurden 50µl Coatingbuffer (50mM Na₂CO₃, 50mM NaHCO₃, pH 9,6) mit einem Gehalt von 250ng des jeweiligen Antikörpers für 3h bei Raumtemperatur inkubiert und dabei der Antikörper an die ELISA-Platten angelagert. Anschließend wurden unspezifische Bindungsstellen durch Inkubation mit 200µl 5x WAPU (12,5ml TritonX100, 62,5ml 1M Tris-HCl pH 7,5, 62,5ml einer 20%igen Gelatinelösung, ad 500ml mit H₂O) für die Dauer von 30 Minuten blockiert.

Die C. *difficile* Stämme 10463, 8864, 1470 und 7002/8 wurden wie in Beispiel 1 beschrieben angezogen, und 50µl-Aliquots des Kultur-Überstands wurden auf die mit den Antikörpern beschichteten Mikrotiterplatten gegeben. Unspezifisch gebundene Proteine wurden durch dreimaliges Waschen mit TBST (150mM NaCl, 10 mM Tris, 0,05% Tween20, pH 8,0) entfernt. Anschließend wurden die gegebenenfalls von dem jeweiligen Antikörper gebundenen Toxine mit 50µl polyklonalem Anti-Toxin-B-Serum (266ng/ml) im Fänger-ELISA detektieren. Die Entwicklung erfolgte über alkalische Phosphatase mit Standard-Verfahren.

Die Ergebnisse dieses ELISA sind in Fig. 5 graphisch dargestellt. Aus Fig. 5 ist ersichtlich, dass jeder der vier verschiedenen *C*. *difficile* Stämme mit den vier eingesetzten Antikörpern ein eigenes, für ihn charakteristisches Reaktionsmuster zeigt, das sich von den Reaktionsmustern der anderen Stämme unterscheidet:
Der Überstand von Stamm VPI-10463 reagiert positiv mit den Antikörpern TGC 35/Gruppe I, TGC 23/Gruppe II und TTC8/Gruppe VI und negativ mit TGC 16/Gruppe IV (siehe Fig. 5A).

Der Überstand von Stamm 8864 reagiert positiv mit den Antikörpern TGC 35/Gruppe I und TGC 16/Gruppe IV und negativ mit TGC 23/Gruppe II und TTC8/Gruppe VI (siehe Fig. 5B).

Der Überstand von Stamm 1470 (Ribotyp 017) reagiert positiv mit den Antikörpern TGC 35/Gruppe I, TGC 23/Gruppe II und TGC 16/Gruppe IV und negativ mit TTC8/Gruppe VI (siehe Fig. 5C).

Der Überstand von Stamm 7002/8 (Ribotyp 027) reagiert positiv mit den Antikörpern TGC 35/Gruppe I, TGC 16/Gruppe IV und TTC8/Gruppe VI und negativ mit TGC 23/Gruppe II (siehe Fig. 5D).

Eine Überprüfung dieser Ergebnisse in Parallelversuchen mit Toxin B von C. *difficile* Stämmen des Ribotyp 027 und verschiedenen Kombinationen von Antikörpern der Antikörpergruppen I bis VI bestätigt, dass TcdB-027 mit Antikörpern der Gruppen I, IV, V und VI immer positiv und mit Antikörpern der Gruppen II und III immer negativ reagiert.

Mit anderen Worten: Das Reaktionsmuster "+--+++" von Toxin B eines unbekannten *C. difficile* Stammes mit den Antikörpern der sechs Antikörpergruppen in der Reihenfolge Gruppe I, Gruppe II, Gruppe III, Gruppe IV, Gruppe V, Gruppe VI erlaubt die Schlussfolgerung, dass es sich bei dem unbekannten *C*. *difficile* Stamm um den bzw. einen Ribotyp-027 handelt, wozu auch die hochvirulenten Stämme *C.d.-027-hv* zählen.

Eine Überprüfung dieser Ergebnisse in Parallelversuchen mit Toxin B von *C. difficile* Stämmen des Ribotyp 017 und verschiedenen Kombinationen von Antikörpern der Antikörpergruppen I bis VI bestätigt, dass TcdB-017 mit Antikörpern der Gruppen I, II und IV immer positiv und mit Antikörpern der Gruppen III, V und VI immer negativ reagiert.

Mit anderen Worten: Das Reaktionsmuster "++-+--" von Toxin B eines unbekannten *C. difficile* Stammes mit den Antikörpern der sechs Antikörpergruppen in der Reihenfolge Gruppe I, Gruppe II, Gruppe III, Gruppe IV, Gruppe V, Gruppe VI erlaubt die Schlussfolgerung, dass es sich bei dem unbekannten *C. difficile* Stamm um den bzw. einen Ribotyp-017 handelt, wozu auch die hochvirulenten Stämme *C.d.-017-hv* zählen.

### Beispiel 4: Identifizierung von C. difficile Ribotypen anhand von Antigen-Antikörper-Reaktions-Referenzmustern

Für die Erstellung der Antigen-Antikörper-Reaktions-Referenzmuster wird wie in Beispiel 3 beschrieben vorgegangen.

Für die Erstellung des Proben-spezifischen Reaktionsmusters wird wie folgt vorgegangen: Der zu untersuchende *C*. *difficile* Stamm wird mit bekannten Verfahren angezogen und der Überstand oder die gereinigten Toxine getestet. Dazu wird jeweils wenigstens ein Antikörper aus wenigstens zwei, vorzugsweise drei oder vier der Gruppen I, II, III, IV, V und VI, z.B. die Antikörper TGC 16, TGC 19, TGC 23, TGC 41, TGC 35 und TTC8 mit dem Fachmann bekannt und geläufigen Verfahren auf eine geeignete Oberfläche, z.B. eine Membran aufgebracht.

Anschließend wird die Probe mit diesen Antikörpern, im folgenden Identifizierungs-Antikörper genannt - in Kontakt gebracht. Geeignete Verfahren hierfür sind dem Fachmann bekannt, beispielsweise der ELISA oder sogenannte "lateral flow"-Verfahren wie das in EP 1143248 beschriebene.

Am Ende der verfahrensspezifischen Reaktionszeit wird bzw. ist jeder Ansatz von nicht gebundenem Probenmaterial befreit (gewaschen).

Um zu detektieren, welcher Identifizierungs-Antikörper mit der Probe eine Antigen-Antikörper-Reaktion zeigt und welcher nicht reagiert hat, wird ein Detektions-Antikörper eingesetzt. Als Detektions-Antikörper können polyklonale Seren gegen Toxin A und Toxin-B (monospezifisch oder kreuzreaktiv) verwendet werden, oder auch Antikörper aus einer oder mehreren der Gruppen I, II, III, IV, V oder VI verwendet werden. Bei der Auswahl der Detektions-Antikörper ist zu beachten, dass sich ihr Zielepitop von den Zielepitopen der Identifizierungs-Antikörper unterscheidet oder grundsätzlich mehrfach in jedem Toxin vorhanden ist, damit der Detektions-Antikörper das Toxin im Verbund mit dem Identifizierungs-Antikörper binden kann. Die Detektions-Antikörper sind mit geeigneten Marker wie z.B. kolloidalem Gold, Kohle- oder Latexpartikeln oder enzymatischen Markern wie HRP (Horse Radish Peroxidase) oder alkalische Phosphatase markiert.

Der Detektions-Antikörper wird entweder vor der Applikation der Probe oder zusammen mit der Probe oder nach Ablauf der verfahrensspezifischen Reaktionszeit zwischen Identifizierungs-Antikörper und Probe zu dem Ansatz gegeben.

Da am Ende der verfahrensspezifischen Reaktionszeit Proben-Toxin, das nicht an die Identifizierungs-Antikörper gebunden ist, und in der Probe eventuell vorhandene kontaminierende Proteine entfernt (abgewaschen) werden, tritt ein Marker-Signal (positives Signal "+") nur auf, wenn ein Verbund (Komplex) aus Identifizierungs-Antikörper, Proben-Toxin und Detektions-Antikörper gebildet worden ist.

Die für jeden Identifizierungs-Antikörper gewonnenen Markersignale "+" oder "-" werden im Reaktionsmuster zusammengestellt. Ein solches Reaktionsmuster kann z.B. aussehen wie Fig. 5A. Der Vergleich mit den Referenzmustern führt in diesem Fall zu dem Schluss (bzw. zu der Verdachtsdiagnose), dass der C. *difficile* Stamm VPI-10463 vorliegt.

Wenn das Reaktionsmuster z.B. aussieht wie Fig. 5B, führt der Vergleich mit den Referenzmustern zu dem Schluss (der Verdachtsdiagnose), dass der *C. difficile* Stamm 8864 vorliegt. Wenn das Reaktionsmuster z.B. aussieht wie Fig. 5C, führt der Vergleich mit den Referenzmustern zu dem Schluss (der Verdachtsdiagnose), dass der *C. difficile* Stamm 1470 bzw. ein *C. difficile* Stamm vom Ribotyp 017 vorliegt. Wenn das Reaktionsmuster z.B. aussieht wie Fig. 5D, führt der Vergleich mit den Referenzmustern zu dem Schluss (der Verdachtsdiagnose), dass der *C. difficile* Stamm 7002/8 bzw. ein *C*. *difficile* Stamm vom Ribotyp 027 vorliegt.

### Beispiel 5: Analyse verschiedener C. difficile Stämme mit Antikörpern der Gruppen I, II, IV, VI

Im folgenden Experiment wurde das erfindungsgemäße Verfahren zur Identifikation und Detektion varianter *C*. *difficile* Stämme anhand einer Reihe von Stämmen mit bekanntem Ribotyp, nämlich 19 Stämmen vom Ribotyp 027 (Lumc-1, Lumc-2, Lumc-3, Lumc-4, Lumc-5, Lumc-6, Lumc-8, Lumc-9, Lumc-10, Lumc-11, Lumc-12, Lumc-13 ,Lumc-14, Lumc-16, Lumc-17, Lumc-19, Lumc-20, Lumc-21, 7002-42), 4 Stämmen vom Ribotyp 017 (Lumc-22, Lumc-23, Lumc-40, Lumc-41), 2 Stämmen vom Ribotyp 001 (7002-29, 7002-43) und ein Stamm vom Ribotyp 014 (Lumc-31), durchgeführt. Als Referenz-Stämme wurden die Stämme VPI-10463, 8864 und 7002/8 verwendet. Antikörper der Gruppen I, II, IV und VI wurden in einem Fänger-ELISA eingesetzt und die resultierenden Reaktionsmuster mit den erstellen Referenzmustern verglichen (s. Beispiel 3).

Die verschiedenen *C*. *difficile* Stämme wurden wie in Beispiel 1 beschrieben angezogen und der Überstand in den sich anschließenden Experimenten verwendet. Als Identifizierungs-Antikörper wurden die Antikörper TGC 35 (Gruppe I), TGC 23 (Gruppe II), TGC 16 (Gruppe IV) und TTC8 (Gruppe VI) wie in Beispiel 3 beschrieben an die ELISA-Platten angelagert. Dabei wurden jeweils 250ng/well TGC 23, 128ng/well TGC 16 und TGC 35, sowie 1µlg/well TTC8 verwendet und die Platten anschließend in PBS gewaschen.

Zur Durchführung des ELISAs wurden die unspezifischen Bindungsstellen der Platten zunächst durch 1h Inkubation bei 37°C in PBS+3% BSA blockiert. Anschließend wurden 50µl/well des Überstandes der *C*. *difficile* Kulturen aufgetragen und die Platten erneut 1h bei 37°C inkubiert. Nicht gebundene Proteine wurden durch dreimaliges Waschen mit PBS+3% BSA entfernt. Als Detektions-Antikörper wurde im Falle von TGC 35, TGC 16 und TGC 23 Biotin-markiertes polyklonales Anti-Toxin-B-Serum (0,32µg/ml) verwendet. Im Falle des Toxin A Nachweises wurde der Antikörper TTC8 in biotinylierter Form auch als Detektions-Antikörper verwendet. Nach erneutem Waschen mit PBS+3% BSA wurden 50µl Streptavidin-HRP (0,1µg/ml, Pierce, Rockford, USA) pro well zugegeben und die Platten 1h bei 37°C inkubiert. Anschließend wurden die Platten mit PBS gewaschen und zur Entwicklung wurden 50µl/well TMB (1-Step Ultra TMB, Pierce, Rockford, USA) verwendet. Die Platten wurden 30min bei Raumtemperatur im Dunkeln inkubiert und die Reaktion anschließend mit 100µl/well 2M H₂SO₄ abgestoppt. Die Auswertung erfolgte bei OD450 gegen einen Leerwert. Als Cut-Off Wert wurde 0,5 festgelegt.

Die Ergebnisse dieses Versuches sind in Fig. 6A und Fig. 6B graphisch dargestellt:
- In 19 verschiedenen Stämmen wurde das Reaktionsmuster "+ - + +" in der Reihenfolge der Gruppen I, II, IV und VI detektiert.
- In 4 verschiedenen C.d.-017-hv-Stämmen wurde das Reaktionsmuster "+++-" in der Reihenfolge der Gruppen I, II, IV und VI detektiert. Die Analyse der 017 Stämme zeigt, dass die Stämme in vitro insgesamt relativ wenig Toxin produzieren (auch das TGC 35 Signal ist verhältnismäßig schwach). Es wird aber deutlich, dass nur bei diesen Stämmen eine spezifische Reaktion zwischen dem Antikörper TGC 23 (DSM ACC 2917) und dem Toxin B-017 stattfindet. Während bei allen anderen Überständen (also allen C.d.-027-hv Stämmen, sowie sämtlichen getesteten Ribotyp 001 Stämmen) die Werte bei der Verwendung des Detektionsantikörpers TGC 23 nicht über 0,4 lagen, lag bei den C.d.-017-hv Stämme die Absorption jeweils deutlich über dem Cut-Off Wert von 0,5.

Die Ergebnisse bestätigen, dass das Antigen-Antikörper-Reaktionsmuster "+ - + +" in der Reihenfolge der Gruppen I, II, IV und VI die Schlußfolgerung zuläßt, dass es sich bei dem untersuchten Stamm um einen C.d.-027-hv Stamm handelt, und dass bei einem Reaktionsmuster "+ + + -" in der Reihenfolge der Gruppen I, II, IV und VI geschlossen werden muss, dass ein C.d.-017-hv Stamm vorliegt.

### Verzeichnis der genannten Literaturstellen:

Moos et al. (2000) "Purification and evaluation of large clostridial cytotoxins that inhibit small GTPases of Rho and Ras subfamilies" Meth Enzymol. 325: 114-125.
Soehn et al. (1998) "Genetic rearrangement in the pathogenicity locus of Clostridium difficile strain 8864 - implications for transcription, expression and enyzmatic activity of toxin A and B" Mol Gen Genet 258: 222-232.
Stubbs et al (1999) "PCR targeted to the 16-23rRNA gene intergenic space region of Clostridium difficile and construction of a library of 116 different PCR ribotypes" J Clin Microbiol 37: 461-463.
von Eichel-Streiber et al. (1995) "Closing in on the toxic domain through analysis of a variant Clostridium difficile cytotoxin B" Mol Microbiol 17: 313-321.

## Patentansprüche

1. In-vitro-Verfahren zum Nachweis und zur Identifikation eines virulenten, LCTs produzierenden, varianten *C*. *difficile* Stammes in einer Probe, **gekennzeichnet durch** die folgenden Verfahrensschritte:
(a) In-Kontakt-bringen einer Körperausscheidungs- und/oder Körpergewebeprobe mit jeweils wenigstens einem Antikörper aus wenigstens drei der Antikörpergruppen Gruppe I, Gruppe II, Gruppe III, Gruppe IV, Gruppe V und Gruppe VI, wobei
Gruppe I die folgenden Antikörper umfasst: monoklonaler Antikörper 2CV (DSM ACC 2321), monoklonaler Antikörper TGC 35 (DSM ACC2918) und pan-TcdB Antikörper,
Gruppe II die folgenden Antikörper umfasst: monoklonale Antikörper TGC23 (DSM ACC2917) und Antikörper, die mit den Toxinen TcdB-10463 und TcdB-017 reagieren, nicht hingegen mit den Toxinen TcdB-8864 und TcdB-027
Gruppe III die folgenden Antikörper umfasst: monoklonale Antikörper TGC41 (DSM ACC2919) und Antikörper, die mit TcdB-10463 reagieren, nicht hingegen mit den Toxinen TcdB-8864, TcdB-017 und TcdB-027
Gruppe IV die folgenden Antikörper umfasst: monoklonale Antikörper TGC 16 (DSM ACC2915) und Antikörper, die mit den Toxinen TcdB-8864, TcdB-017 und TcdB-027 reagieren, nicht hingegen mit dem Toxin TcdB-10463
Gruppe V die folgenden Antikörper umfasst: monoklonale Antikörper TGC 19 (DSM ACC2916) und Antikörper, die mit den Toxinen TcdB-8864 und TcdB-027 reagieren, nicht hingegen mit den Toxinen TcdB-10463 und TcdB-017
Gruppe VI die folgenden Antikörper umfasst: monoklonale Antikörper TTC8 (DSM ACC 2322) und pan-TcdA Antikörper;
und wobei gilt:
TcdB-10463 steht für Toxin B des *C*. *difficile* Stammes VPI-10463,
TcdB-8864 steht für Toxin B des *C*. *difficile* Stammes 8864,
TcdB-017 steht für Toxin B von *C*. *difficile* Stämmen mit Ribotyp 017, insbesondere für Toxin B von *C*. *difficile* Stamm 1470,
TcdB-027 steht für Toxin B von *C*. *difficile* Stämmen mit Ribotyp 027, insbesondere für Toxin B von *C*. *difficile* Stamm 7002/8
(b) Detektion der Antikörperreaktion und Erstellen des Reaktionsmusters, wobei jedem in (a) eingesetzten Antikörper jeder Antikörpergruppe I bis VI ein Positiv-Symbol (z.B."+") für eine detektierte positive Antikörperreaktion und ein Negativ-Symbol (z.B."-") für eine detektierte negative Antikörperreaktion zugeordnet wird, und
(c) Vergleich des in (b) gewonnenen Reaktionsmusters mit Referenzmustern, die **dadurch** gewonnenen werden, dass von den im Stand der Technik bekannten und im Nachweistest auf Präsenz zu überprüfenden *C. difficile* Stämmen Rx₁, Rx₂, Rxᵢ wenigstens eines ihrer jeweiligen großen clostridialen Toxine (LCT) LCT-Rx₁, LCT-Rx₂, LCT-Rxᵢ in einem Reaktionstest mit den in Schritt (a) gewählten Antikörpern aus den wenigstens drei der Antikörper-Gruppen I bis VI eingesetzt wird, und dass die für jedes LCT und die wenigstens drei der Antikörper-Gruppen I bis VI erhaltenen Reaktionsergebnisse derart musterartig registriert werden, dass jeder Antikörpergruppe entweder ein Positiv-Symbol (z.B."+") im Fall einer detektierten positive Antikörperreaktion oder ein Negativ-Symbol (z.B. "-") im Fall einer detektierten negative Antikörperreaktion zugeordnet wird,
(d) Bewertung einer Übereinstimmung zwischen dem in (b) gewonnenen Reaktionsmusters mit einem Referenzmuster als Indiz dafür, dass in der Probe der *C*. *difficile* Stamm des betreffenden Referenzmusters vorliegt.

2. Verfahren nach Anspruch 1, **dadurch gekennzeichnet, dass** die Antikörper monoklonale Antikörper sind.

3. Verfahren nach Anspruch 1 oder 2, **dadurch gekennzeichnet, dass** der wenigstens eine Antikörper aus Gruppe IV der monoklonale Antikörper TGC 16 (DSM ACC2915) ist.

4. Verfahren nach einem der Ansprüche 1 bis 3, **dadurch gekennzeichnet, dass** der wenigstens eine Antikörper aus Gruppe V der monoklonale Antikörper TGC 19 (DSM ACC2916) ist.

5. Verfahren nach einem der Ansprüche 1 bis 4, **dadurch gekennzeichnet, dass** der wenigstens eine Antikörper aus Gruppe VI der monoklonale Antikörper TTC8 (DSM ACC 2322) ist.

6. Verfahren nach einem der Ansprüche 1 bis 5 **dadurch gekennzeichnet, dass** der wenigstens eine Antikörper aus Gruppe I der monoklonale Antikörper 2CV (DSM ACC 2321) oder der monoklonale Antikörper TGC 35 (DSM ACC2918) ist.

7. Verfahren nach einem der Ansprüche 1 bis 6 **dadurch gekennzeichnet, dass** der wenigstens eine Antikörper aus Gruppe II der monoklonale Antikörper TGC23 (DSM ACC2917) ist.

8. Verfahren nach einem der Ansprüche 1 bis 7 **dadurch gekennzeichnet, dass** der wenigstens eine Antikörper aus Gruppe III der monoklonale Antikörper TGC41 (DSM ACC2919) ist.

9. Verfahren nach einem der Ansprüche 1 bis 8, **dadurch gekennzeichnet, dass**
in Schritt (a) die Probe mit jeweils einem Antikörper aus jeder der Gruppen I, IV und VI in Kontakt gebracht wird, und dass
in Schritt (b) eine positive Reaktion der Antikörper aus den Gruppen I, IV und VI das Vorliegen eines hochvirulenten, varianten *C*. *difficile* Stammes anzeigt.

10. Verfahren nach einem der Ansprüche 1 bis 8 **dadurch gekennzeichnet, dass**
in Schritt (a) die Probe mit jeweils wenigstens einem Antikörper aus jeder der Antikörpergruppen Gruppe II, Gruppe IV und Gruppe VI in Kontakt gebracht wird, und dass
in Schritt (b) eine positive Reaktion der Antikörper aus Gruppe IV und Gruppe VI und eine negative Reaktion des/der Antikörper(s) aus Gruppe II das Vorliegen des hochvirulenten *C*. *difficile* Stamms Ribotyps 027, PFGE-NAP1, REA-B1 und Toxinotyp III anzeigen.

11. Verfahren nach Anspruch 10, **dadurch gekennzeichnet, dass**
in Schritt (a) die Probe zusätzlich mit jeweils wenigstens einem Antikörper der Antikörpergruppe I und/oder der Antikörpergruppe III und/oder der Antikörpergruppe V in Kontakt gebracht wird, und dass
in Schritt (b) zusätzlich eine positive Reaktion des/der Antikörper(s) aus Gruppe I und Gruppe V und eine negative Reaktion des/der Antikörper(s) aus Gruppe III das Vorliegen des hochvirulenten *C*. *difficile* Stamms Ribotyps 027, PFGE-NAP1, REA-B1 und Toxinotyp III anzeigen.

12. Verfahren nach einem der Ansprüche 1 bis 8 **dadurch gekennzeichnet, dass**
in Schritt (a) die Probe mit jeweils wenigstens einem Antikörper aus jeder der Antikörpergruppen Gruppe II, Gruppe IV und Gruppe VI in Kontakt gebracht wird, und dass
in Schritt (b) eine positive Reaktion der Antikörper aus Gruppe II und Gruppe IV und eine negative Reaktion des/der Antikörper(s) aus Gruppe VI das Vorliegen des hochvirulenten *C*. *difficile* Stamms Ribotyp 017, Toxinotyp VIII und Serotyp F anzeigen.

13. Verfahren nach Anspruch 12, **dadurch gekennzeichnet, dass**
in Schritt (a) die Probe zusätzlich mit jeweils wenigstens einem Antikörper der Antikörpergruppe I und/oder der Antikörpergruppe III und/oder der Antikörpergruppe V in Kontakt gebracht wird, und dass
in Schritt (b) zusätzlich eine positive Reaktion des/der Antikörper(s) aus Gruppe I und eine negative Reaktion der Antikörper aus Gruppe III und Gruppe V das Vorliegen des hochvirulenten *C*. *difficile* Stamms Ribotyp 017, Toxinotyp VIII und Serotyp F anzeigen.

14. Verwendung des monoklonalen Antikörpers, der von der bei der DSMZ, Braunschweig, Deutschland unter der Nummer DSM ACC2916 (TGC 19) hinterlegten Hybridomazellinie produziert wird, in einem Verfahren nach einem der Ansprüche 1 bis 13.

15. Verwendung des monoklonalen Antikörpers, der bei der DSMZ, Braunschweig, Deutschland unter der Nummer DSM ACC2917 (TGC 23) hinterlegten Hybridomazellinie produziert wird, in einem Verfahren nach einem der Ansprüche 1 bis 13.

16. Verwendung des monoklonalen Antikörpers, der bei der DSMZ, Braunschweig, Deutschland unter der Nummer DSM ACC2915 (TGC 16) hinterlegten Hybridomazellinie produziert wird, in einem Verfahren nach einem der Ansprüche 1 bis 13, insbesondere nach einem der Ansprüche 10 bis 13.

17. Verwendung des monoklonalen Antikörpers, der bei der DSMZ, Braunschweig, Deutschland unter der Nummer DSM ACC2918 (TGC 35) hinterlegten Hybridomazellinie produziert wird, in einem Verfahren nach einem der Ansprüche 1 bis 13.

## Claims

1. In-vitro- method for detecting and identifying a virulent LCT producing variant C. *difficile* strain in a sample, **characterized by** the following method steps:
(a) bringing a sample of excreta and/or tissue from a body into contact with at least one antibody from each of at least three of the antibody groups group I, group II, group III, group IV, group V and group VI, wherein:
group I comprises the following antibodies: monoclonal antibody 2CV (DSM ACC 2321), monoclonal antibody TGC 35 (DSM ACC 2918) and pan-TcdB antibodies;
group II comprises the following antibodies: monoclonal antibody TGC 23 (DSM ACC 2917) and antibodies that react with toxins TcdB-10463 and TcdB-017, but not with toxins TcdB-8864 and TcdB-027;
group III comprises the following antibodies: monoclonal antibody TGC 41 (DSM ACC 2919) and antibodies that react with TcdB-10463, but not with toxins TcdB-8864, TcdB-017 and TcdB-027;
group IV comprises the following antibodies: monoclonal antibody TGC 16 (DSM ACC 2915) and antibodies that react with toxins TcdB-8864, TcdB-017 and TcdB-027, but not with toxin TcdB-10463;
group V comprises the following antibodies: monoclonal antibody TGC 19 (DSM ACC 2916) and antibodies that react with toxins TcdB-8864 and TcdB-027, but not with toxins TcdB-10463 and TcdB-017;
group VI comprises the following antibodies: monoclonal antibody TTC8 (DSM ACC 2322) and pan-TcdA antibodies;
and wherein:
TcdB-10463 stands for toxin B from *C*. *difficile* strain VPI-10463,
TcdB-8864 stands for toxin B from *C*. *difficile* strain 8864,
TcdB-017 stands for toxin B from *C*. *difficile* strains with ribotype 017, in particular for toxin B from *C*. *difficile* strain 1470,
TcdB-027 stands for toxin B from *C*. *difficile* strains with ribotype 027, in particular for toxin B from *C*. *difficile* strain 7002/8
(b) detecting the antibody reaction and constructing a reaction pattern whereby each antibody of each antibody group I to VI used in (a) is assigned a positive symbol (for example "+") for a positive detected antibody reaction and a negative symbol (for example "-") for a negative detected antibody reaction; and
(c) comparing the reaction pattern obtained in (b) with reference patterns which are obtained from *C*. *difficile* strains Rx₁, Rx₂, Rxᵢ which are known in the art and are to be tested for their presence in the detection test by carrying out a reaction test between at least one of their large clostridial toxins (LCT) LCT-Rx₁, LCT-Rx₂, LCT-Rxᵢ and the antibodies from at least three of the antibody groups I to VI selected in step (a), and wherein the reaction results obtained for each LCT and the at least three antibody groups from groups I to VI are recorded in a pattern so that each antibody group is assigned either a positive symbol (for example "+") in the event of a positive detected antibody reaction or a negative symbol (for example "-") in the event of a negative detected antibody reaction;
(d) assessing a match between the reaction pattern obtained in (b) and a reference pattern as indicative of the presence of the *C*. *difficile* strain of the reference pattern concerned in the sample.

2. The method according to claim 1, **characterized in that** the antibodies are monoclonal antibodies.

3. The method according to claim 1 or 2, **characterized in that** the at least one antibody from group IV is the monoclonal antibody TGC 16 (DSM ACC 2915).

4. The method according to any one of claims 1 to 3, **characterized in that** the at least one antibody from group V is the monoclonal antibody TGC 19 (DSM ACC 2916).

5. The method according to any one of claims 1 to 4, **characterized in that** the at least one antibody from group VI is the monoclonal antibody TTC8 (DSM ACC 2322).

6. The method according to any one of claims 1 to 5, **characterized in that** the at least one antibody from group 1 is the monoclonal antibody 2CV (DSM ACC 2321) or the monoclonal antibody TGC 35 (DSM ACC 2918).

7. The method according to any one of claims 1 to 6, **characterized in that** the at least one antibody from group II is the monoclonal antibody TGC 23 (DSM ACC 2917).

8. The method according to any one of claims 1 to 7, **characterized in that** the at least one antibody from group III is the monoclonal antibody TGC 41 (DSM ACC 2919).

9. The method according to any one of claims 1 to 8, **characterized in that** in step (a) the sample is brought into contact with at least one antibody from each of groups I, IV and VI; and that in step (b) a positive reaction of the antibodies from groups I, IV and VI indicates the presence of a highly virulent variant *C*. *difficile* strain.

10. The method according to any one of claims 1 to 8, **characterized in that** in step (a) the sample is brought into contact with at least one antibody from each of antibody groups II, IV and VI; and that in step (b) a positive reaction of the antibodies from groups IV and VI and a negative reaction of the antibody(ies) from group II indicates the presence of the highly virulent variant *C*. *difficile* strain ribotype 027, PFGE-NAP1, REA-B1 and toxinotype III.

11. The method according to claim 10, **characterized in that** in step (a) the sample is additionally brought into contact with at least one antibody from group I and/or III and/or V; and that in step (b), a positive reaction of the antibody(ies) from group I and group V and a negative reaction of the antibody(ies) from group III additionally indicates the presence of the highly virulent variant *C*. *difficile* strain ribotype 027, PFGE-NAP1, REA-B1 and toxinotype III.

12. The method according to any one of claims 1 to 8, **characterized in that** in step (a) the sample is brought into contact with at least one antibody from each of antibody groups II, IV, and VI; and that in step (b) a positive reaction of the antibodies from groups II and IV and a negative reaction of the antibody(ies) from group VI indicates the presence of the highly virulent variant *C*. *difficile* strain ribotype 017, toxinotype VIII and serotype F.

13. The method according to claim 12, **characterized in that** in step (a) the sample is additionally brought into contact with at least one antibody from antibody group I and/or from antibody group V; and **in that** in step (b) a positive reaction of the antibody(ies) from group I and a negative reaction of the antibodies from group III and group V additionally indicates the presence of the highly virulent variant *C*. *difficile* strain ribotype 017, toxinotype VIII and serotype F.

14. Use of the monoclonal antibody produced from the hybridoma cell line deposited with the DSMZ, Braunschweig, Germany with accession number DSM ACC 2916 (TGC 19), in a method according to any one of claims 1 to 13.

15. Use of the monoclonal antibody produced from the hybridoma cell line deposited with the DSMZ, Braunschweig, Germany with accession number DSM ACC 2917 (TGC 23), in a method according to any one of claims 1 to 13.

16. Use of a monoclonal antibody produced from the hybridoma cell line deposited with the DSMZ, Braunschweig, Germany with accession number DSM ACC 2915 (TGC 16), in a method according to any one of claims 1 to 13, in particular according to any one of claims 10 to 13.

17. Use of a monoclonal antibody produced from the hybridoma cell line deposited with the DSMZ, Braunschweig, Germany with accession number DSM ACC 2918 (TGC 35), in a method according to any one of claims 1 to 13.

## Revendications

1. Procédé in vitro de détection et d'identification dans un échantillon d'une souche de *C*. *difficile* variante, produisant des LCT et virulente, **caractérisé par** les étapes suivantes :
(a) mise en contact d'un échantillon d'excrétion corporelle et/ou d'un échantillon de tissu corporel avec au moins un anticorps de respectivement au moins trois des groupes d'anticorps groupe I, groupe II, groupe III, groupe IV, groupe V et groupe VI,
le groupe I comprenant les anticorps suivants : anticorps monoclonal 2CV (DSM ACC 2321), anticorps monoclonal TGC 35 (DSM ACC2918) et anticorps pan-TcdB,
le groupe II comprenant les anticorps suivants : anticorps monoclonal TGC23 (DSM ACC2917) et des anticorps qui réagissent avec les toxines TcdB-10463 et TcdB-017, mais par contre ne réagissent pas avec les toxines TcdB-8864 et TcdB-027
le groupe III comprenant les anticorps suivants : anticorps monoclonal TGC41 (DSM ACC2919) et des anticorps qui réagissent avec TcdB-10463, mais par contre ne réagissent pas avec les toxines TcdB-8864, TcdB-017 et TcdB-027
le groupe IV comprenant les anticorps suivants : anticorps monoclonal TGC 16 (DSM ACC2915) et des anticorps qui réagissent avec les toxines TcdB-8864, TcdB-017 et TcdB-027, mais qui par contre ne réagissent pas avec la toxine TcdB-10463
le groupe V comprenant les anticorps suivants : anticorps monoclonal TGC 19 (DSM ACC2916) et des anticorps qui réagissent avec les toxines TcdB-8864 et TcdB-027, mais qui par contre ne réagissent pas les toxines TcdB-10463 et TcdB-017
le groupe VI comprenant les anticorps suivants : anticorps monoclonal TTC8 (DSM ACC 2322) et anticorps pan-TcdA ;
étant précisé que
TcdB-10463 signifie toxine B de la souche C. *difficile* VPI-10463,
TcdB-8864 signifie toxine B de la souche C. *difficile* 8864,
TcdB-017 signifie toxine B de souches *C*. *difficile* de ribotype 017, notamment la toxine B de la souche *C*. *difficile* 1470,
TcdB-027 signifie toxine B de souches *C*. *difficile* de ribotype 027, notamment la toxine B de la souche *C. difficile* 7002/8
(b) détection de la réaction de l'anticorps et établissement d'un modèle de réaction, un symbole positif (par exemple "+") pour une réaction d'anticorps détectée positive et un symbole négatif (par exemple "-") pour une réaction d'anticorps détectée négative étant attribué à chaque anticorps de chaque groupe d'anticorps I à IV utilisé à l'étape (a), et
(c) comparaison du modèle de réaction obtenu en (b) avec des modèles de référence qui sont obtenues par le fait qu'au moins l'une des grandes toxines clostridiales (LCT) LCT-Rx₁, LCT-Rx₂, LCT-Rxᵢ des souches C. *difficile* Rx₁, Rx₂, Rxᵢ connues de l'état de la technique et dont la présence est à vérifier dans le test de détection, est utilisée dans un test de réaction avec les anticorps choisis à l'étape (a) parmi les au moins trois groupes d'anticorps I à VI, et que les résultats de réaction obtenus pour chaque LCT et les au moins trois des groupes d'anticorps I à VI sont enregistrés à la façon d'un modèle de telle sorte qu'à chaque groupe d'anticorps est attribué soit un symbole positif (par exemple "+") dans le cas d'une réaction d'anticorps détectée positive soit un symbole négatif (par exemple "-") dans le cas d'une réaction d'anticorps détectée négative,
(d) évaluation d'une concordance entre le modèle de réaction obtenu en (b) et un modèle de référence comme étant un d'indice que la souche de *C*. *difficile* du modèle de référence correspondant se trouve dans l'échantillon.

2. Procédé selon la revendication 1, **caractérisé en ce que** les anticorps sont des anticorps monoclonaux.

3. Procédé selon la revendication 1 ou 2, **caractérisé en ce que** le au moins un anticorps du groupe IV et l'anticorps monoclonal TGC 16 (DSM ACC2915).

4. Procédé selon l'une des revendications 1 à 3, **caractérisé en ce que** le au moins un anticorps du groupe V est l'anticorps monoclonal TGC 19 (DSM ACC2916).

5. Procédé selon l'une des revendications 1 à 4, **caractérisé en ce que** le au moins un anticorps du groupe VI est l'anticorps monoclonal TTC8 (DSM ACC 2322).

6. Procédé selon l'une des revendications 1 à 5, **caractérisé en ce que** le au moins un anticorps du groupe I est l'anticorps monoclonal 2CV (DSM ACC 2321) ou l'anticorps monoclonal TGC 35 (DSM ACC2918).

7. Procédé selon l'une des revendications 1 à 6, **caractérisé en ce que** le au moins un anticorps du groupe II est l'anticorps monoclonal TGC23 (DSM ACC2917).

8. Procédé selon l'une des revendications 1 à 7, **caractérisé en ce que** le au moins un anticorps du groupe III est l'anticorps monoclonal TGC41 (DSM ACC2919).

9. Procédé selon l'une des revendications 1 à 8, **caractérisé en ce que**
à l'étape (a), l'échantillon est mis en contact avec un anticorps de chacun des groupes I, IV et VI, et **en ce que**
à l'étape (b) une réaction positive des anticorps des groupes I, IV et VI indique la présence d'une souche variante hautement virulente de *C*. *difficile.*

10. Procédé selon l'une des revendications 1 à 8, **caractérisé en ce que**
à l'étape (a), l'échantillon est mis en contact avec au moins un anticorps de chacun des groupes d'anticorps groupe II, groupe IV et groupe VI, et **en ce que**
à l'étape (b) une réaction positive des anticorps du groupe IV et du groupe VI et une réaction négative du/des anticorps du groupe II indiquent la présence de la souche hautement virulente *C*. *difficile* de ribotype 027, PFGE-NAP1, REA-B1 et de toxinotype III.

11. Procédé selon la revendication 10, **caractérisé en ce que**
à l'étape (a), l'échantillon est mis en contact en outre avec au moins un anticorps du groupe d'anticorps I et/ou du groupe d'anticorps III et/ou du groupe d'anticorps V, et **en ce que**
à l'étape (b), de plus, une réaction positive du/des anticorps du groupe I et du groupe V et une réaction négative du/des anticorps du groupe III indiquent la présence de la souche hautement virulente de *C*. *difficile* de ribotype 027, PFGE-NAP1, REA-B1 et de toxinotype III.

12. Procédé selon l'une des revendications 1 à 8, **caractérisé en ce que**
à l'étape (a), l'échantillon est mis en contact avec au moins un anticorps de chacun des groupes d'anticorps groupe II, groupe IV et groupe VI, et **en ce que**
à l'étape (b) une réaction positive des anticorps du groupe II et du groupe IV et une réaction négative du/des anticorps du groupe VI indiquent la présence de la souche hautement virulente *C*. *difficile* de ribotype 017, de toxinotype VIII et de sérotype F.

13. Procédé selon la revendication 12, **caractérisé en ce que**
à l'étape (a), l'échantillon est en outre mis en contact avec au moins un anticorps du groupe d'anticorps I et/ou du groupe d'anticorps III et/ou du groupe d'anticorps V, et **en ce que**
à l'étape (b), en plus, une réaction positive du/des anticorps du groupe I et une réaction négative des anticorps du groupe III et du groupe V indiquent la présence de la souche hautement virulente *C*. *difficile* de ribotype 017, de toxinotype VIII et de sérotype F.

14. Utilisation dans un procédé selon l'une des revendications 1 à 13 de l'anticorps monoclonal produit par la lignée cellulaire d'hybridome déposée auprès du DSMZ, Braunschweig, Allemagne, sous le numéro DSM ACC2916 (TGC 19).

15. Utilisation dans un procédé selon l'une des revendications 1 à 13 de l'anticorps monoclonal produit par la lignée cellulaire d'hybridome déposée auprès du DSMZ, Braunschweig, Allemagne, sous le numéro DSM ACC2917 (TGC 23).

16. Utilisation dans un procédé selon l'une des revendications 1 à 13, en particulier selon l'une des revendications 10 à 13, de l'anticorps monoclonal produit par la lignée cellulaire d'hybridome déposée auprès du DSMZ, Braunschweig, Allemagne, sous le numéro DSM ACC2915 (TGC 16).

17. Utilisation dans un procédé selon l'une des revendications 1 à 13 de l'anticorps monoclonal produit par la lignée cellulaire d'hybridome déposée auprès du DSMZ, Braunschweig, Allemagne, sous le numéro DSM ACC2918 (TGC 35).
